# EUROPEAN PATENT SPECIFICATION

(11) **EP 1 276 892 B1**
(45) Date of publication and mention of the grant of the patent: **24.11.2004**
(21) Application number: 01925789.8
(22) Date of filing: 29.03.2001
(51) Int. Cl.: C12Q 1/00

(54) **A BACTERIAL TWO-HYBRID SYSTEM FOR PROTEIN INTERACTION SCREENING**
EIN ZWEIHYBRID-SCREENINGSYSTEM IN BAKTERIEN FÜR PROTEINWECHSELWIRKUNG
UN SYSTEME BACTERIEN A DEUX HYBRIDES DANS LE CRIBLAGE DE L'INTERACTION PROTEINE-PROTEINE

(30) Priority: 29.03.2000 US 192886 P
(43) Date of publication of application: 22.01.2003
(73) Proprietor: INSTITUT PASTEUR, 75015 Paris (FR); CENTRE NATIONAL DE LA RECHERCHE SCIENTIFIQUE (CNRS), 75794 Paris Cedex 16 (FR); Hybrigenics S.A., 75012 Paris (FR)
(72) Inventor: KARIMOVA, Gouzel, F-75006 Paris (FR); LADANT, Daniel, F-94230 Cachan (FR); ULLMANN, Agnès, F-75016 Paris (FR); SELIG, Luc, F-94220 Charenton le Pont (FR); LEGRAIN, Pierre, F-75015 Paris (FR)
(74) Representative: Warcoin, Jacques
(86) International application number: PCT/IB2001/000773
(87) International publication number: WO 2001/073108

(56) References cited:
- WO-A-99/28746
- KARIMOVA ET AL: "A bacterial two-hybrid system based on a reconstituted signal transduction pathway" PROCEEDINGS OF THE NATIONAL ACADEMY OF SCIENCES OF USA, NATIONAL ACADEMY OF SCIENCE. WASHINGTON, US, vol. 95, May 1998 (1998-05), pages 5752-5756, XP002100623 ISSN: 0027-8424 cited in the application

## Description

### BACKGROUND OF THE INVENTION

The present invention concerns the construction of new strains useful in a method for selecting a molecule, a kit therefor, a method for screening a molecule, a kit therefor, and a signal amplification system comprising a bacterial multi-hybrid system. More particularly, the present invention relates to improvements to the technique referred as a multi-hybrid system described in PCT application No. WO 99/28746, published on June 10, 1999. This technology is also described in *Proc. Natl. Acad. Sci. USA* in 1998,95(10) pages 5752-5756 (Karimova et al.).

A novel bacterial two-hybrid system that allows an easy *in* vivo screening and selection of functional interactions between two proteins has recently been described. This genetic system is based on the reconstitution of an artificial cAMP signal transduction pathway in an *Escherichia coli* adenylate cyclase deficient strain (*cya*). It takes advantage of the modular structure of the catalytic domain of *Bordetella pertussis* adenylate cyclase, which consists of two complementary fragments. *Bordetella pertussis* adenylate cyclase: a toxin with multiple talents *Trends in Microbiol.* 7, 172-176). When they are expressed separately in *E. coli,* they can not be converted to an active enzyme unless interacting polypeptides are genetically fused to these fragments.

In the bacterial two-hybrid system, interaction between the two chimeric proteins results in functional complementation between the two adenylate cyclase fragments and restoration of enzymatic activity. The resulting cAMP synthesis triggers the expression of several *E. coli* resident genes, thus giving rise to a selectable phenotype.

By design, this bacterial two-hybrid system is taking place in an *E. coli cya* strain, i.e., lacking its endogenous adenylate cyclase. Numerous *E. coli* cya strains harboring point mutations, deletions or insertions within the *cya* gene (adenylate cyclase structural gene) have been described. Using a modified phosphomycin selection procedure, the DHP1 strain was isolated. This strain is a spontaneous *cya* derivative of DH1 (F-, *glnV44(AS), recA1, endA1, gyrA96 (Nal*^{*r*}*), thil, hsdR17, spoT1, rfbD1*). Functional complementation between hybrid proteins appeared to be much more efficient in DHP1 than in other *cya* strains that were tested, possibly, because of the higher stability of chimeric proteins.

It has been discovered, however, that DHP1 exhibits a high frequency (at about 10⁻⁶) of spontaneous reversion of the Cya⁻ phenotype towards a Cya⁺ phenotype. This characteristic is, therefore, limiting the utility of DHP1 in large scale screening applications, such as a library screening when a great number of transformed cells have to be analyzed.

There exists a need in the art for bacterial strains that can be employed in the methods and kits described in published PCT application No. 99/28746. The new bacterial strains should exhibit a Cya phenotype, allowing efficient functional complementation between standard hybrid proteins, and be suitable for use in large scale analysis of protein-protein interactions.

### SUMMARY OF THE INVENTION

This invention aids in fulfilling these needs in the art. More particularly, it is an aim of this invention to provide new tools for using the bacterial multi-hybrid systems, methods, and kits disclosed in published PCT application WO 99/28746.

More particularly, this invention relates to improvements in the methods and kits described in published PCT Application WO 99/28746. In particular, this invention provides improved bacterial strains for use in the methods and kits. The bacterial strains are selected from the group consisting of strain **BTH101** having C.N.C.M. Deposit Accession No. I-2309 and strain **DHM1** having C.N.C.M. Deposit Accession No. I-2310.

In one embodiment, this invention provides a signal amplification system as described in WO 99/28746 using strain **BTH101** or strain **DHM1** of this invention.

In another embodiment, this invention provides a method of selecting a molecule of interest as described in WO 99/28746. The method utilizes the signal amplification system described in WO 99/28746 in which the strain **BTH101** or the strain **DHM1** is employed.

In a further embodiment, this invention provides a kit for selecting a molecule of interest. The kit is as described in WO 99/28746 and utilizes strain **BTH101** or strain **DHM1** of this invention.

This invention also provides a method of screening for a substance capable of stimulating or inhibiting the interaction between a target ligand and a molecule of interest as described in WO 99/28746. The method utilizes strain **BTH101** or strain **DHM1** of this invention.

Further, this invention provides a kit for screening for a substance capable of stimulating or inhibiting the interaction between a target ligand and a molecule of interest. The kit is as described in WO 99/28746 and utilizes strain **BTH101** or strain **DHM1** of this invention.

This invention also provides strain **BTH101** having C.N.C.M. Deposit Accession No. I-2309.

In addition, this invention provides strain **DHM1** having C.N.C.M. Deposit Accession No. I-2310.

This invention also provides a DNA library comprising a collection of vectors transformed in a bacterial multi-hybrid system of the invention using strain **BTH101** or strain **DHM1.** Each plasmid can contain a polynucleotide coding for the molecule of interest fused to a polynucleotide coding for the first or the second fragment of an enzyme. The polynucleotide coding for the molecule of interest can be, for example, a fragment of genomic DNA of *Helicobacter pylori*.

In another embodiment, this invention provides an *H. pylori* DNA library, HGX BHP₃, having C.N.C.M. Deposit Accession No. I-2367.

In summary, the present invention relates to the use of new strains, which are characterized as **DHM1** and **BTH101,** and plasmids contained in *E. coli* XL-1/pUT18, XL-1/pUT18C, XL1-1/pT25, and XL-1/pKT25.

### BRIEF DESCRIPTION OF THE DRAWINGS

Fig. 1 shows the results of zip-zip interaction in different *E. coli cya* strains. β-galactosidase activity is shown for three different strains, DHM1, BHT101, and DHP1.
Fig. 2 shows the results of interaction in different strains. β-galactosidase activity is shown for the strains DHM1 and BTH101.
Fig. 3 is a map of plasmid pUT18C NewSfi.
Fig. 4 is a map of plasmid pKT25 NewSfi.

### DETAILED DESCRIPTION OF THE INVENTION

Most biological processes involve specific protein-protein interactions. General methodologies to identify interacting proteins or to study these interactions have been extensively developed. Among them, the yeast two-hybrid system currently represents the most powerful *in vivo* approach to screen for polypeptides that could bind to a given target protein. It utilizes hybrid genes to detect protein-protein interactions by means of direct activation of a reporter-gene expression.

In essence, the two putative protein partners are genetically fused to the DNA-binding domain of a transcription factor and to a transcriptional activation domain, respectively. A productive interaction between the two proteins of interest will bring the transcriptional activation domain in the proximity of the DNA-binding domain and will trigger directly the transcription of an adjacent reporter gene (usually *lacZ* or a nutritional marker) giving a screenable phenotype. There is evidence that the transcription can be activated through the use of two functional domains of a transcription factor: a domain that recognizes and binds to a specific site on the DNA and a domain that is necessary for activation.

Published PCT application WO 99/28746 describes a signal amplification system comprising a bacterial multi-hybrid system of at least two chimeric polypeptides containing:
(a) a first chimeric polypeptide corresponding to a first fragment of an enzyme;
(b) a second chimeric polypeptide corresponding to a second fragment of an enzyme or a modulating substance capable of activating said enzyme, wherein the first fragment is fused to a molecule of interest and the second fragment or the modulating substance is fused to a target ligand, and wherein the activity of the enzyme is restored by the *in vivo* interaction between the said molecule of interest and the said target ligand and wherein a signal amplification is generated.

The signal amplification system is useful in a method of selecting a molecule of interest, which is capable of binding to a target ligand, wherein the interaction between the molecule of interest and the target ligand is detected with the signal amplification system by generating a signal amplification and triggering transcriptional activation.

Published PCT application WO 99/28746 also describes a kit for selecting a molecule of interest, wherein the kit comprises:
(a) the signal amplification system;
(b) an *E. coli* strain or in any bacterial strain deficient in endogenous adenylate cyclase or any other eukaryotic cell; and
(c) a medium allowing the detection of the complementation. The medium is selected from the group consisting of indicator or selective medium as minimal medium supplemented with lactose or maltose as unique carbon source, medium with antibiotics, medium to visualize fluorescence, conventional medium, and medium that allows the sorting by the presence of the phage receptor.

The kit described in WO 99/28746 for selecting the molecule of interest can also comprise:
(a) the signal amplification system, wherein the molecule of interest is a mutant molecule compared to the known wild type molecule;
(b) the signal amplification system, wherein the molecule of interest is the known wild type molecule as the control;
(c) *E. coli* strain or in any bacterial strain deficient in endogenous adenylate cyclase or any other eukaryotic cell;
(d) a medium allowing the detection of the complementation selected from the group consisting of indicator or selective medium as minimal medium supplemented with lactose or maltose as unique carbon source, medium with antibiotics, medium to visualize fluorescence, conventional medium, and medium that allows the sorting by the presence of the phage receptor for each signal amplification system; and
(e) means for detecting whether the signal amplification system with the mutant molecule is enhanced or inhibited with respect to the signal amplification system with wild type.

In addition, published PCT application WO 99/28746 describes a method of screening for a substance capable of stimulating or inhibiting the interaction between a target ligand and a molecule of interest. The stimulating or the inhibiting activity is detected with the signal amplification system according by means of generating an amplification and respectively of triggering or of abolishing transcriptional activation. The signal amplification and the triggered or abolished transcriptional activation are compared with those obtained from an identical signal amplification system without any substance. The method of screening for a substance capable of stimulating or inhibiting the interaction between a target ligand and a molecule of interest can be performed in an *E. coli* strain or in any bacterial strain deficient in endogenous adenylate cyclase or any other eukaryotic cell.

Further, published PCT application WO 99/28746 describes a kit for screening for the substance capable of stimulating or inhibiting the interaction between a target ligand and a molecule of interest. The kit comprises:
(a) the signal amplification system with the substance capable of stimulating or inhibiting the interaction between a target ligand and a molecule of interest;
(b) the signal amplification system without any substance as the control;
(c) an *E. coli* strain or any bacterial strain deficient in endogenous adenylate cyclase or any other eukaryotic cell;
(d) a medium allowing the detection of the complementation selected from the group consisting of indicator plate or selective medium as minimal medium supplemented with lactose or maltose as unique carbon source, medium with antibiotics, medium to visualize fluorescence, conventional medium, and medium that allows the sorting by the presence of the phage receptor; and
(e) means for detecting whether the signal amplification system with the substance is enhanced or inhibited with respect to the signal amplification system without any substance.

The original *Escherichia coli cya* strain, DHP1, used in the bacterial two-hybrid system and other methods and kits described in published PCT application WO 99/28746 was a spontaneous *cya* mutant that was isolated as phosphomycin resistant. Although complementation worked efficiently in the DHP1 strain, it had a tendency to revert to a Cya+ phenotype at a high frequency, which precluded its utilization in large scale screening.

New *cya* strains have been constructed. These new strains of this invention (i) should not revert and (ii) should exhibit an efficient complementation. Two such strains were selected, **DHM1** and **BTH101,** and are described below. Bacterial strains and plasmids used are listed in Table 1.

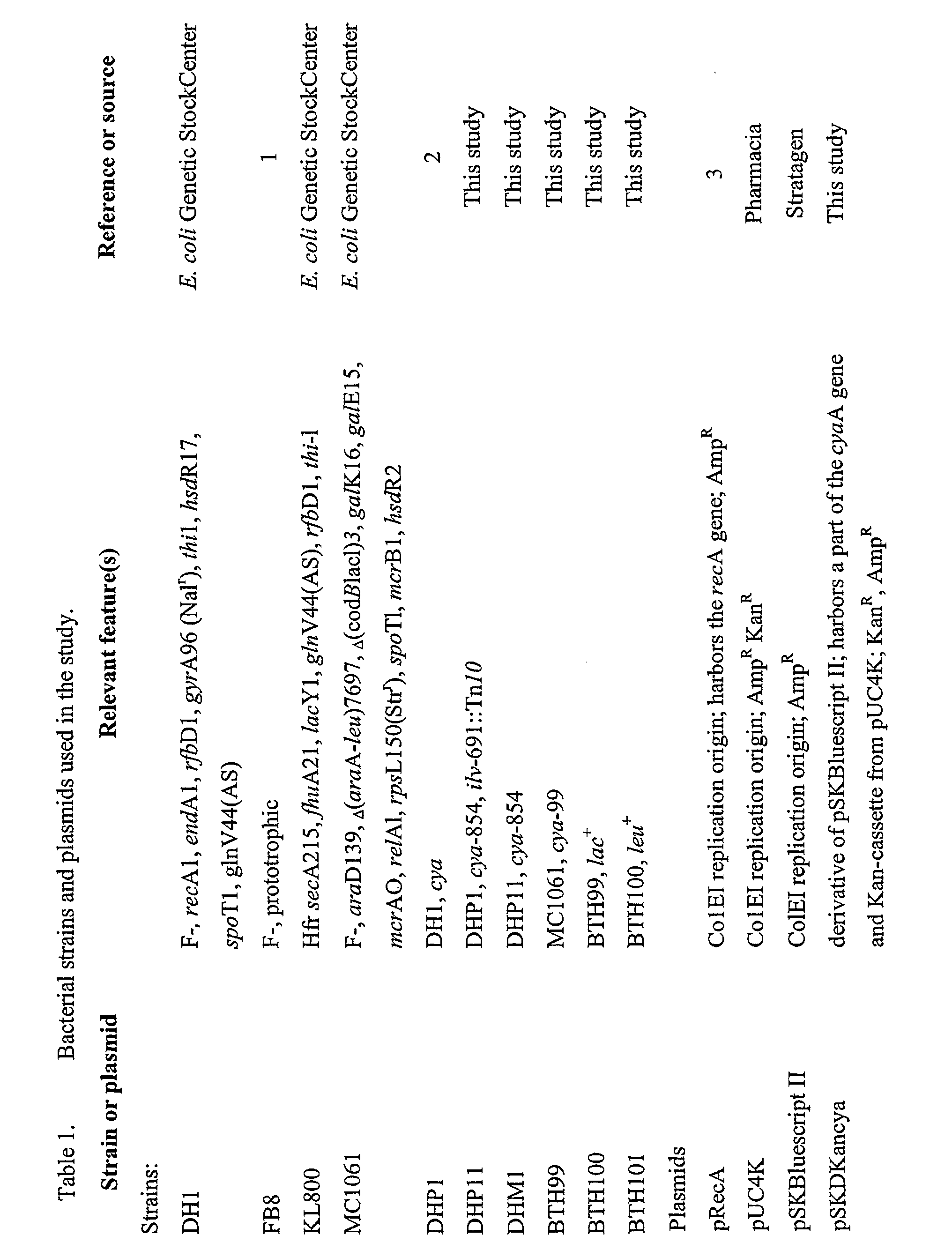

1. Uzan, M. and Danchin A., 1978. Correlation between the serine sensitivity and the derepressibility of the *ilv* genes in Escherichia coli *rel*A-mutants. *Mol Gen Genet.,* 165:21-30.
2. Karimova, G., Pidoux, J., Ullmann, A. & Ladant, D., 1988. A bacterial two-hybrid system based on reconstituted signal transduction pathway. *Proc. Natl. Acad. Sci. U.S.A.* 95:5752-5756.
3. Sancar, A., Rupp, W.D., 1979. Physical map of the *recA* gene. *Proc. Natl. Acad. Sci. U.S.A.* 76:3144-3148.

The two strains of this invention, **DHM1** and **BTH101,** can be employed in the methods and kits described in published PCT application WO 99/28746. The entire disclosure of published PCT application WO 99/28746 is relied upon. The terms used herein have the same definitions as in WO 99/28746.

The prior bacterial strain **DHP1** and the new strains **DHM1** and **BTH101** will now be described in greater detail.

### 1. DHP1

**DHP1** strain described in published PCT application WO 99/28746 is an adenylate cyclase deficient (*cya*) derivative of DH1 (F-, *glnV44(AS), recA1, endA1, gyrA96, (Nal*^{*r*}*), thil, hsdR17, spoT1, rfbD1*) (25), and was isolated using phosphomycin as a selection antibiotic (Alper, M. D. & Ames, B. N. (1978) *J. Bacteriol.* 133, 149-57). Growth media used were the rich medium LB or the synthetic medium M63 (Miller, J. H. (1972) *Experiments in molecular genetics* (Cold Spring Harbor Laboratory, Cold Spring Harbor, N.Y.)) supplemented with 1 % carbon source. Antibiotic concentrations were ampicillin 100 mg/ml and chloramphenicol 30 mg/ml. Screening for the ability to ferment sugars was performed either on MacConkey agar plates containing 1 % maltose, or on LB plates containing 40 mg/ml X-Gal (5-Bromo-4-chloro-3-indolyl-β-D-galactopyranoside) and 0.5 mM IPTG (Isopropyl-β-D-thiogalactopyranoside).

### 2. DHM1: F-, recA1, endA1, gyrA96Nal^{r}), thi1, hsdR17, spoT1, rfbD1, glnV44(AS), cya-854

**DHM1** strain is a derivative of DHP that harbors a small deletion within the cya gene (*cya*-854) that was introduced by P1 transduction (Miller, 1972). For this purpose, a P1 vir phage lysate was prepared on the donor strain FB8 *cya*-854 *ilv* 691::Tn*10* (kind gift of M. Perrote). This strain has a transposon Tn*10* insert in close linkage to *cya* (Wanner, 1986). This linkage was used to transfer the *cya*-85s4 mutation into DHP1 that harbors a plasmid, pRecA, a pBR322 derivative that encodes the *recA* gene of *E. coli* and restores a Rec⁺ phenotype (G. Karimova, unpublished results). The resulting Tc^{R} (tetracycline resistant) transductants were tested for the presence of the mutant allele *cya-854,* a 200-base-pair deletion within the *cya* gene (Glaser, P., Roy A, Danchin, A., 1989). This was carried out by restreaking the cells on MacConkey/maltose indicator plates with tetracycline (25 µg/ml). The transductants harboring the *cya*-854 mutation could be readily distinguished from those harboring the original *cya* mutation from DHP1 as they do not revert (that is no Cya⁺ cells -red phenotype- could be detected in patches of a large number of colonies), whereas the transductants with the original DHP1 *cya* mutation reverted with high frequency as indicated by the appearance of red Cya⁺ cells within patches of a large number of colonies. The frequency of co-transduction of two markers, *ilv*-691::Tn*10* and *cya*-854, was about 30 %. One such transductant was selected and called **DHP11.**

**DHM1** strain was constructed by P1 transduction of the wild type allele of *ilv* from *E. coli* K-12 FB8 into DHP11 harboring pRecA. The transductants were selected on minimal medium without added amino acids. After reisolation of the resulting transductants, the presence of the *cya*-857 mutation and the loss of Tn*10* (Tc^{s}-sensitivity) were verified by plating on MacConkey/maltose and LB containing Tc (25 µg/ml) plates. To cure pRecA plasmid, the transductants DHM1(pRecA) were grown in L-broth overnight and then spread on MacConkey/maltose plates. To identify DHM1 cells that lost pRecA, 100 singles colonies were restreaked on the two plates: one LB plate containing ampicillin (Amp, 100 µg/ml) and one MacConkey/maltose plate without antibiotic. Plates were grown for 24 hours at 37°C. Several Amp-sensitive clones were picked from the MacConkey/maltose plate, and after restreaking and further testing for complementation, one clone, called DHM1, was selected.

To examine the frequency of Lac⁺ and Mal⁺ revertants among DHM1 cells, an overnight culture of DHM1 (10 ml) in LB was collected by centrifugation, washed 3 times by B63 medium, and plated on minimal medium with X-gal supplemented by maltose or lactose as unique carbon sources. Frequencies of Lac⁺ and Mal⁺ clones were, respectively, 10⁻⁷ and 10⁻⁹. Note that these Lac⁺ and Mal⁺ revertants are not Cya⁺ revertants but arise from cAMP/CAP independent mutations within the *lac* promoter or cAMP-independent CAP mutations (CRP^{*}).

### 3. BTH101: F⁻, galE15, galK16, rpsL1 (Str^{r}), hsdR2, mcrA1, mcrB1, cya-99

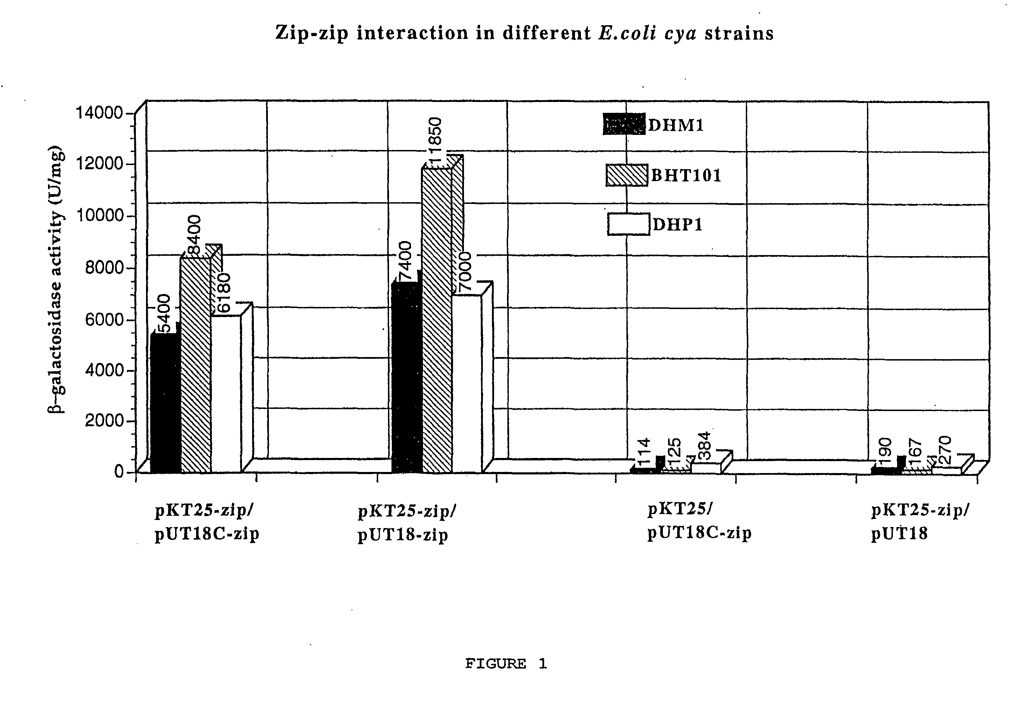

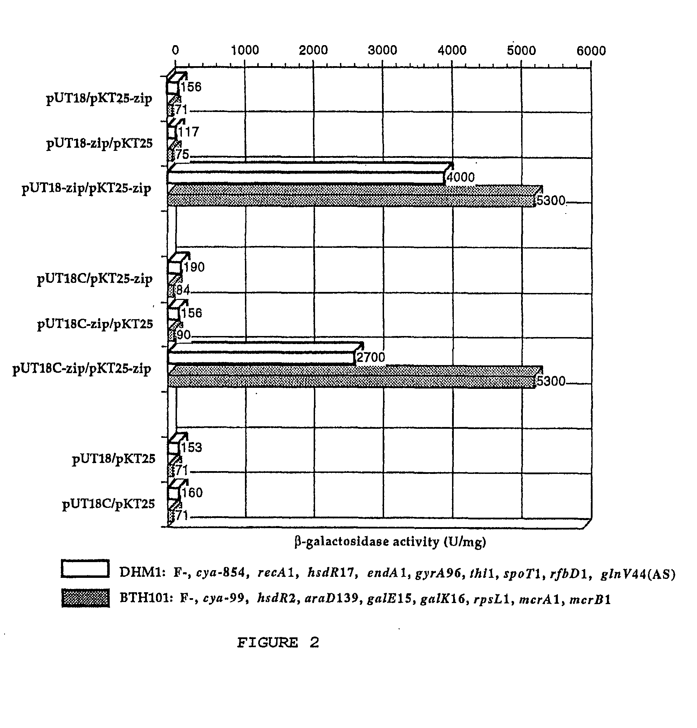

**BTH101** strain is a derivative of MC1061 that was constructed by conjugation and P1 transduction techniques (Miller, 1972). While an attempt was made to introduce a precise deletion within the *cya* gene, we isolated a spontaneous *cya* mutant of MC1061, **BTH99,** that most likely harbors a deletion within the *cya* gene as it reverts to Cya⁺ (in fact Lac⁺ or Mal⁺ phenotype) with a very low frequency. This mutant was obtained as follows. First, a plasmid was constructed, pSKDKancya, by inserting within pSKBluescript II vector (Stratagene) - between the *Xho*I and *Xba*I sites - the following fragments of DNA: a 1.5 kb fragment of *E. coli* chromosomal DNA immediately upstream from the *cyaA* gene, a chloramphenicol-resistant cassette, a 1.5 kb fragment of *E. coli* chromosomal DNA immediately downstream from the *cyaA* gene, and a kanamycin-resistant cassette (Karimova, unpublished data). Plasmid pSKDKancya was transformed into the donor strain Hfr KL800 and the resulting transformants were mated with the recipient strain MC1061 (Streptomycin resistant) for 30 min at 37° C. Aliquots of a mating mixture were plated on MacConkey maltose indicator plates supplemented by Kan (50 µg/ml) and Str (100 µg/ml, to conterselect donor cells). The recipient Kan^{R} and Str^{R} cells were then mixed with a lambda vir phage stock in order to select *cya* derivatives (*E. coli cya* cells are resistant to λ) and re-plated on MacConkey maltose. Several λ^{R}, *cya* derivatives were isolated. None of them harbored the intended *cya* deletions, but several of the clones obtained exhibited a stable *cya* phenotype (i.e., they reverted towards Lac⁺ or Mal⁺ phenotypes with a very low frequency). To identify clones, which lost the plasmid, the reisolated recombinants were subsequently plated on LB-Kan and MacConkey maltose indicator plates. One of such *cya,* Kan^{s}-sensitive clones, **BTH99**, was kept for further study as it exhibited good complementation capabilities in bacterial two-hybrid assays with standard complementing plasmids (pKT25-zip/pUT18-zip, see Fig. 1 and Fig. 2). The mutation cya-99 of BTH99 is currently under characterization.

**BTH101** is a derivative of BTH99 that was created by two successive P1 transductions. First, the wild type *lac* operon was transduced from FB8, the transductants were selected on minimal plates supplemented by lactose in the presence of 1mM cAMP. Lac⁺ clones **(BTH100)** were reisolated and tested for the presence of *cya-99* by using MacConkey/lactose indicator plates with and without cAMP. BTH101 strain was made by PI transduction of the wild type allele of *ilv* (from FB8) into BTH100. The resulting clones were selected on minimal medium without amino acids. One such clone, BTH101, was kept for further characterization.

Plasmids and strains useful for practicing this invention have been deposited at Collection Nationale de Cultures de Microorganismes in Paris, France as follows:

| Plasmid | Deposit Date | Accession No. |
|---|---|---|
| XL-1/pUT18 | November 25, 1998 | I-2092 |
| XL-1/pUT18C | November 25, 1998 | I-2093 |
| XL-1/pT25 | November 25, 1998 | I-2094 |
| XL-1/pKT25 | November 25, 1998 | I-2095 |
| DHM1 | September 10, 1999 | I-2310 |
| BTH101 | September 10, 1999 | I-2309 |
| *H. pylori* | December 14, 1999 | I-2367 |
| library | | |
| HGX BHP₃ | | |

Plasmid pKT25 (3445-bp) is a derivative of the low copy vector pSU40 (expressing a kan amycin resistance selectable marker) that encodes the T25 fragment. It was constructed as follows: a 1044-bp *Hind*III-*Eco*RI, fragment of pT25 was first subcloned into pSU40 linearized with *Hind*III and *Eco*RI, resulting in pKT25L. pKT25 was generated from pKT25L by deleting a 236-bp *Nhe*I-*Hind*III fragment.

**Plasmid pUT18 (3023-bp)** is a derivative of the high copy number vector pUC19 (expressing an ampicillin resistance selectable marker and compatible with pT25 or pKT25) that encodes the T18 fragment (amino acids 225 to 399 of CyaA). In a first step, we constructed plasmid pUC19L by inserting a 24-bp double-stranded oligonucleotide (5'-ATTCATCGATATAACTAAGTAA-3' [SEQ ID No. 1]) and its complementary sequence) between the *EcoRI* and *Nde*I sites of pUC 19. Then, a 534-bp fragment harboring the T18 open reading frame was amplified by PCR (using appropriate primers and pT18 as target DNA) and cloned into pUC19L digested by *Eco*RI and *Cla*I (the appropriate restriction sites were included into the PCR primers). In the resulting plasmid, pUT18, the T18 open reading frame is fused in frame downstream of the multicloning site of pUC19. This plasmid is designed to create chimeric proteins in which a heterologous polypeptide is used to the N-terminal end of T18 (see map).

Plasmid **pUT18C** (3017-bp) is a derivative of pUC19 (expressing an ampicillin resistance selectable marker and compatible with pT25 or pKT25) that encodes the T18 fragment. It was constructed by subcloning the same 534-bp PCR-amplified fragment harboring the T18 open reading frame described above into pUC19L linearized by *Hind*III and *Pst*I (the appropriate restriction sites were included into the PCR primers). In the resulting plasmid, pUT18C, the T18 open reading frame is fused in frame upstream of the multicloning site of pUC19L. This plasmid is designed to create chimeric proteins in which a heterologous polypeptide is fused to the C-terminal end of T18 (see map).

Plasmid **pKT25-zip** (3556-bp) is a derivative of pKT25 that was constructed by inserting a DNA fragment (PCR-amplified using appropriate primers) encoding the leucine zipper region of GCN4 into **pKT25** cleaved by *Kpn*I, as described above.

Plasmid **pUT18-zip** (3125-bp) is a derivative of pUT18 that was constructed by inserting a 114bp DNA fragment (PCR-amplified using appropriate primers) encoding the leucine zipper region of GCN4 into pUT18 linearized by *Kpn*I and *Eco*RI.

Plasmid **pUT18C-zip** (3119-bp) is a derivative of pUT18C that was constructed by inserting the same 114-bp DNA fragment encoding the GCN4 leucine zipper described above into pUT18 linearized by *Kpn*I and *Eco*RI.

This invention will be described in greater detail with reference to the following examples.

### EXAMPLE 1

To examine the frequency of Lac+ and Mal+ revertants among BTH 10 cells, an overnight culture of BTH101 (10 ml) in LB was collected by centrifugation, washed 3 times by B63 medium, and plated on minimal medium with X-gal supplemented by maltose or lactose as unique carbon sources. Frequencies of Lac⁺ and Mal⁺ clones were, respectively, 10⁻⁸ and 10⁻⁹.

### EXAMPLE 2

To test the efficiency of complementation in the bacterial two-hybrid system, the different strains, **DHP1, DHM1,** and **BTH101,** were co-transformed with the various couples of plasmid as indicated in Figures 1 and 2. The β-galactosidase activity expressed by each co-transformant was determined on liquid culture as previously described (Karimova *et al*., 1998). Data of Figures 1 and 2 correspond to two different sets of totally independent experiments.

### EXAMPLE 3

### Vector construction

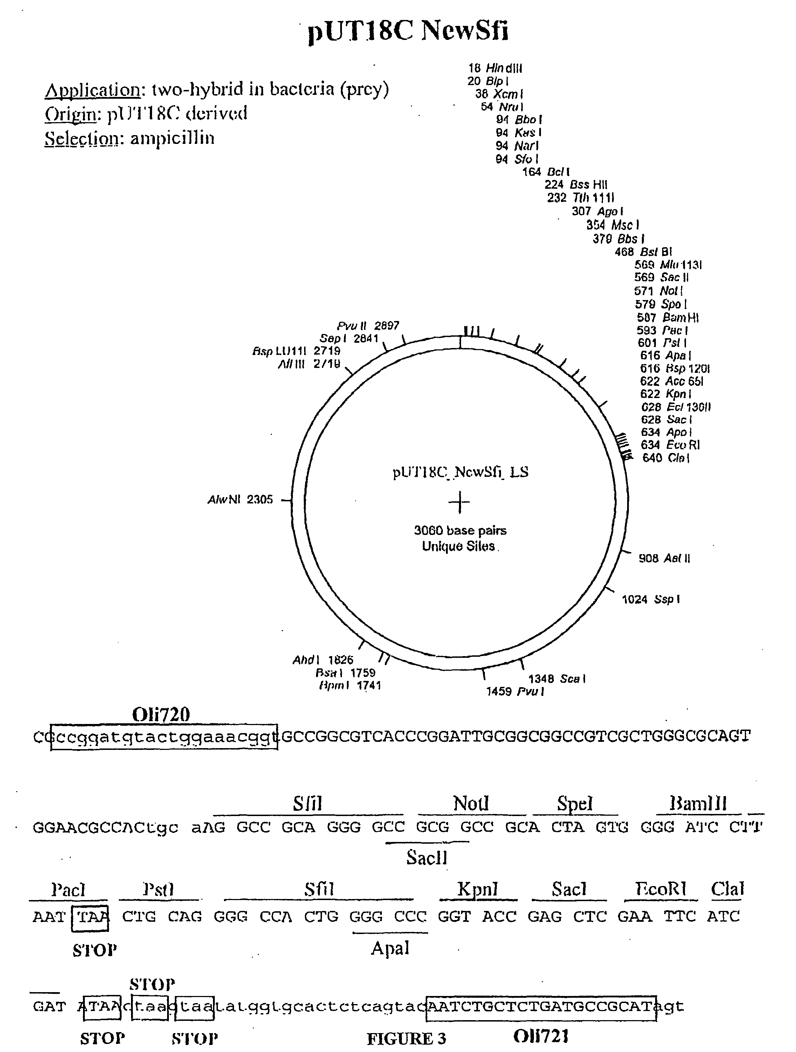

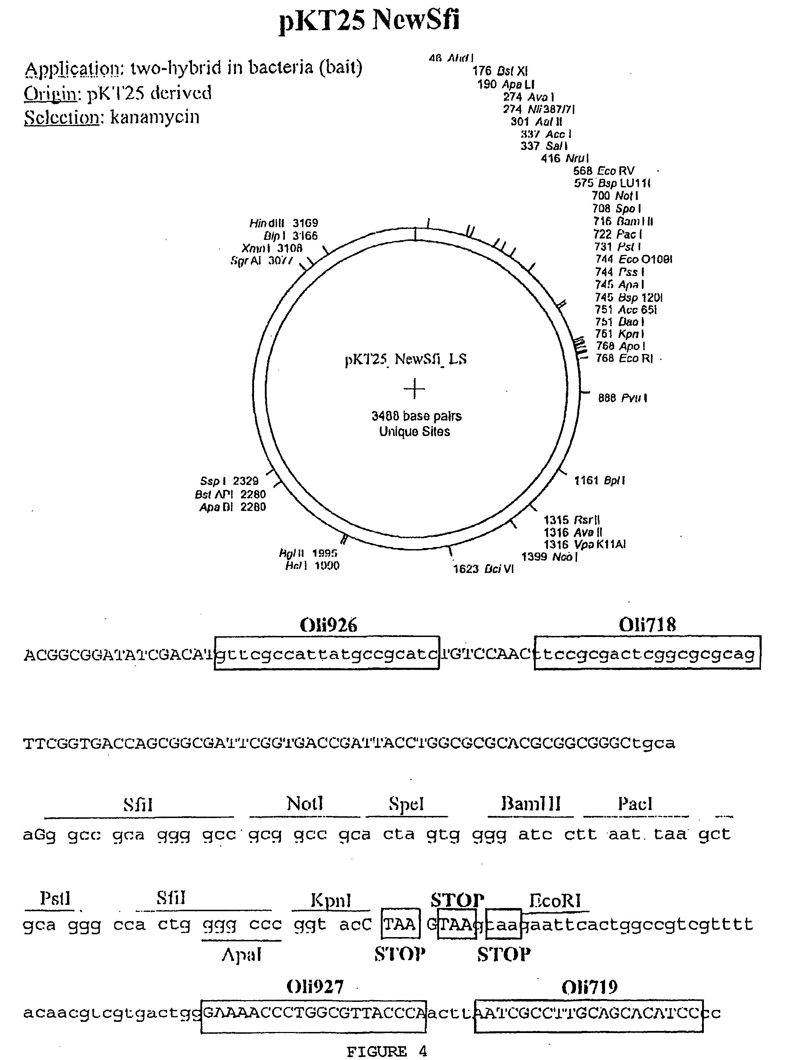

pUT18C is a high-copy number plasmid since it contains the ColEl origin of replication. It was, therefore, used as the prey vector. A new multi-cloning site was created by ligating a long oligonucleotide within the *Kpn*I - *Pst*I sites. The sequences of the top and bottom oligonucleotides were as follows: AGGCCGCAGGGGCCGCGGCC GCACTAGTGGGGATCCTTAATTAACTGCAGGGGCCACTGGGGCCCGGTAC [SEQ ID No. 2] and CGGGCCCCAGTGGCCCCTGCAGTTAATTAA GGATCCCCACTAGTGCGGCCGCGGCCCCTGCGGCCTTGCA [SEQ ID No. 3], respectively. Both oligos were annealed and the resulting double-stranded oligo was ligated into previously digested pUT18C vector. pKT25 NewSFI, a derivative of pKT25, was used as the bait vector. It contains a multi-cloning site similar to the one of pUT18C NewSFI and was constructed with the top AGGGCCGCAGGGGCCGCGGC CGCACTAGTGGGGATCCTTAATTAAGCTGCAGGGCCACTGGGGCCCGGTAC [SEQ ID No. 4] and bottom CGGGCCCCAGTGGCCCTGCAGCTTAATTAA GGATCCCCACTAGTGCGGCCGCGGCCCCTGCGGCCCTTGCA [SEQ ID No. 5] oligos. Maps of these two vectors can be found in Figs. 3 and 4.

7.4 x 10⁶ independent colonies were recovered after electroporation of the ligation products. Out of the 192 randomly chosen colonies, 85 (44 %) contained a plasmid with an insert according to a PCR screening experiment and were subsequently sequenced. Seventy nine sequences were exploitable. 41 (52 %) of the insert-containing plasmids were found in the sense orientation. No obvious bias of cloning was observed from the calculation of the start positions of the inserts. The average size of the 79 inserts was not determined.

### EXAMPLE 4

### Library construction

The method used to clone genomic fragments from *Helicobacter pylori* into pUT18CNS has been described elsewhere (WO 99/28746). Briefly, pUT18CNS was BamHI digested and filled in with a guanosine nucleotide to prevent self-religation of the vector. Nebulized genomic DNA from *H. pylori* was blunt-ended using a cocktail of Mung Bean Nuclease, T4 Polymerase and Klenow enzymes. Inserts were further ligated with adapters into linearized pUT18CNS plasmid. DH10B (Gibco BRL) electro-competent cells were transformed with ligation products and plated on LB + ampicillin. One hundred and ninety two colonies were randomly chosen to estimate the actual number of plasmids with inserts and to detect an eventual bias during the cloning step. These colonies were used as template in PCR experiments using the 720 and 721 oligos. PCR products were further sequenced using the same oligos. Sequences were treated with BLAST software. Remaining colonies were harvested and pooled. 20 ml were aliquoted with glycerol or DMSO in 1ml cryotubes and stored at - 80°C. Remaining of the pool was aliquoted in 50ml Falcon tubes and centrifuged. Supernatants were removed and tubes were stored at - 80°C. Library DNA was extracted using a Qiagen Maxi-Prep kit.

### EXAMPLE 5

### Bait construction

pKT25NS was digested with the *Bam*HI and *Pst*I restriction enzymes. The vector was further dephosphorylated using Calf Intestine Phosphatase. The urease accessory protein UreH (HP0067) has been previously used in a genome-wide approach of *Helicobacter pylori* two-hybrid in yeast screens (WO 99/28746). The insert corresponding to the full-length coding sequence was *Bam*HI *- Pst*I subcloned from pAS2ΔΔ-HP0067 into newly prepared pKT25 NewSFI.

### EXAMPLE 6

### Library screening

DHM1 was used as the *cya*^{*-*} host strain. Electro-competent DHM1 cells were transformed with pKT25 NewSFI-HP0067 and plated on LB + kanamycin. pKT25 NewSFI-HP0067 cells were subsequently rendered electro-competent and transformed with 1:1 of diluted *H. pylori* library (40ng). Transformed cells were incubated 1 hour at 37°C after adding 1 ml of SOC medium and were further washed with 1 ml M9 medium. Following a 1/10000^{th} dilution, cells were plated on LB + kanamycin + ampicillin for counting of double-transformants. Remaining of the transformed cells was plated either undiluted on 9 M63 + maltose + X-Gal + IPTG + kanamycin + ampicillin plates or diluted 1/10^{th} on 9 plates of the same medium or diluted 1/100^{th} on 10 plates of the same medium.

5 x 10⁷ double transformants were obtained following transformation of the DHM1/pKT25 NewSFI-HP0067 with 40 ng of library DNA. About 5 x 10⁶, 5 x 10⁵ and 5 x 10⁴ bacteria were, therefore, plated on the M63 screening plates, when no, 1/10^{th} or 1/100th dilutions were performed, respectively. During a 15 day growth period, 18 positive clones were selected, 3 in the 0 dilution condition, 11 in the 1/10^{th} dilution condition, and 4 in the 1/100^{th} dilution condition.

### EXAMPLE 7

### Prey isolation and identification

Following growth and blue coloration at 30°C, positive clones were further streaked on M63 + maltose + X-Gal + IPTG + kanamycin + ampicillin plates to isolate positive clones from the background negative colonies. Plasmid mini-preps (Qiagen) following overnight culture in LB + kanamycin were made to recover the prey plasmids. Prey inserts were further sequenced using the 720 and 721 primers. BLAST homology was performed on these sequences.

All XX positive clones were sequenced. Clone identification is shown in Table 2.

**TABLE 2**

| Bait | Dilution | Clone | Strand | Pos. | ORF | nt/AUG | Osize | Phase |
|---|---|---|---|---|---|---|---|---|
| HP0067 | 0 | 2 | C | 74170 | HP0069 | 41 | 765 | IF |
| HP0067 | 0 | 3 | C | 74182 | HP0069 | 29 | 765 | IF |
| HP0067 | 0 | 4 | W | 1566571 | HP1493 | 30 | 612 | OOF |
| HP0067 | 10 | 1 | C | 117917 | HP0109 | 308 | 1863 | IF |
| HP0067 | 10 | 2 | W | 692044 | HP0645 | 1681 | 1683 | OOF |
| HP0067 | 10 | 3 | ND | ND | HP 1493 | ND | ND | ND |
| HP0067 | 10 | 4 | W | 1566580 | HP1493 | 39 | 612 | OOF |
| HP0067 | 10 | 5 | C | 488358 | HP0466 | 320 | 768 | IF |
| HP0067 | 10 | 7 | C | 74170 | HP0069 | 41 | 765 | IF |
| HP0067 | 10 | 8 | C | 74194 | HP0069 | 17 | 765 | IF |
| HP0067 | 10 | 9 | C | 143584 | HP0132 | 11 | 1368 | IF |
| HP0067 | 10 | 10 | W | 1566580 | HP1493 | 39 | 612 | OOF |
| HP0067 | 10 | 11 | W | 1566576 | HP1493 | 35 | 612 | IF |
| HP0067 | 10 | 12 | C | 74173 | HP0069 | 38 | 765 | IF |
| HP0067 | 100 | 1 | W | 503238 | HP0480 | 611 | 1800 | IF |
| HP0067 | 100 | 2 | W | 364123 | HP0354 | 1574 | 1857 | IF |
| HP0067 | 100 | 3 | C | 1e+06 | HP1422 | 2021 | 2763 | IF |
| HP0067 | 100 | 4 | C | 1261589 | HP1190 | 386 | 1329 | IF |
| ND: Not Determined | | | | | | | | |

When sorted according to the position of the first nucleotide of the insert, the results in Table 3 are obtained:

**TABLE 3**

| Bait | Dilution | Clone | Strand | Pos. | ORF | nt/AUG | Osize | Phase |
|---|---|---|---|---|---|---|---|---|
| HP0067 | 0 | 2 | C | 74170 | HP0069 | 41 | 765 | IF |
| HP0067 | 10 | 7 | C | 74170 | HP0069 | 41 | 765 | IF |
| HP0067 | 10 | 12 | C | 74173 | HP0069 | 38 | 765 | IF |
| HP0067 | 0 | 3 | C | 74182 | HP0069 | 29 | 765 | IF |
| HP0067 | 10 | 8 | C | 74194 | HP0069 | 17 | 765 | IF |
| HP0067 | 10 | 1 | C | 117917 | HP0109 | 308 | 1863 | IF |
| HP0067 | 10 | 9 | C | 143584 | HP0132 | 11 | 1368 | IF |
| HP0067 | 100 | 2 | W | 364123 | HP0354 | 1574 | 1857 | IF |
| HP0067 | 10 | 5 | C | 488358 | HP0466 | 320 | 768 | IF |
| HP0067 | 100 | 1 | W | 503238 | HP0480 | 611 | 1800 | IF |
| HP0067 | 10 | 2 | W | 692044 | HP0645 | 1681 | 1683 | OOF |
| HP0067 | 100 | 4 | C | 1261589 | HP1190 | 386 | 1329 | IF |
| HP0067 | 100 | 3 | C | 1492663 | HP1422 | 2021 | 2763 | IF |
| HP0067 | 0 | 4 | W | 1566571 | HP1493 | 30 | 612 | OOF |
| HP0067 | 10 | 11 | W | 1566576 | HP1493 | 35 | 612 | IF |
| HP0067 | 10 | 4 | W | 1566580 | HP1493 | 39 | 612 | OOF |
| HP0067 | 10 | 10 | W | 1566580 | HP1493 | 39 | 612 | OOF |
| HP0067 | 10 | 3 | ND | ND | HP1493 | ND | ND | ND |

Five out of the 18 clones correspond to another urease accessory protein, ureF (HP0069), which was also found in the corresponding yeast two-hybrid screen of PCT WO 99/28746. Out of these 5 clones, 4 independent fusions (different start positions of the inserts) were found. All of the inserts were found to be in frame with T18. Five out of the 18 clones correspond to HP 1493, a predicted coding region of *H. pylori,* which has no known homology with any other sequence deposited in public gene databases. Out of the 5 clones, at least 3 independent fusions were found. Two of these 3 independent fusions were found out of frame with T18. This prey was not found in the corresponding yeast two-hybrid screen (WO 99/28746). It might correspond to a novel protein partner of ureH, which can not be isolated with the yeast two-hybrid technology.

The present invention utilizes the signal amplification system in *Escherichia coli* described in WO 99/28746, in which the proteins of interest are genetically fused to two complementary fragments of the catalytic domain of *Bordetella pertussis* adenylate cyclase. *B. pertussis* produces a calmodulin dependent adenylate cyclase toxin encoded by the *cyaA* gene.

The catalytic domain is located within the first 400 amino acids of this 1706 residue-long protein.

The catalytic domain can be proteolytically cleaved into two complementary fragments, T25 and T18, that remain associated in the presence of CaM in a fully active ternary complex. In the absence of CaM, the mixture of the two fragments did not exhibit detectable activity suggesting that the two fragments are not able to reassociate to yield basal CaM-independent activity.

The two complementary fragments, T25 and T18, that are both necessary to form an active enzyme, in the presence of CaM when expressed in *E. coli* as separated entities, are unable to recognize each other and cannot reconstitute a functional enzyme. However, when T25 and T18 are fused to peptides or proteins that are able to interact, heterodimerization of these chimeric polypeptides results in a functional complementation between the adenylate cyclase fragments.

When expressed in an adenylate cyclase deficient *E. coli* strain (*E. coli* lacks CaM or CaM-related proteins), the T25 and T18 fragments fused to putative interacting proteins reassociate and lead to cAMP synthesis.

Interaction between a target ligand and a molecule of interest results in functional complementation between the two adenylate cyclase fragments leading to cAMP synthesis, which in turn can trigger the expression of several resident genes. Using this assay, one can select specific clones expressing a protein that interacts with a given target by a simple genetic screening.

The present invention provides a signal amplification system comprising a bacterial multi-hybrid system, and more preferably a two-hybrid system, of at least two chimeric polypeptides containing a first chimeric polypeptide corresponding to a first fragment of an enzyme, and a second chimeric polypeptide corresponding to a second fragment of an enzyme or a modulating substance capable of activating said enzyme wherein the first fragment is fused to a molecule of interest and the second fragment or the modulating substance is fused to a target ligand and wherein the activity of the enzyme is restored by the *in vivo* interaction between the said molecule of interest and the said target ligand and wherein a signal amplification is generated; and wherein signal amplification is performed in an *E. coli* strain selected from the group consisting of strain **BTH101** having C.N.C.M. Deposit Accession No. I-2309 and strain **DHM1** having C.N.C.M. Deposit Accession No. I-2310.

According to one embodiment of the invention, the enzyme can be selected from the group consisting of adenylate cyclase and guanylate cyclase from any origin. Any origin refers to *Bordetella species* or any other organism that produces this type of enzyme. In one specific illustration, the enzyme is the catalytic domain of *Bordetella* adenylate cyclase (CyaA) located within the first 400 amino acid residues of the adenylate cyclase enzyme as described in WO 99/28746.

The present invention also concerns a first fragment and a second fragment, which are any combination of fragments from the same enzyme, which *in vitro* functionally interact with the natural activator of said enzyme by restoring its activity.

According to one embodiment of the invention the first and the second fragments are selected from the following group described in WO 99/28746.
(a) a fragment T25 corresponding to amino acids 1 to 224 of CyaA and a fragment T18 corresponding to amino acids 225 to 399 of CyaA;
(b) a fragment corresponding to amino acids 1 to 224 of CyaA and a fragment corresponding to amino acids 224 to 384 of CyaA;
(c) a fragment corresponding to amino acids 1 to 137 of CyaA and a fragment corresponding to amino acids 138 to 400 of CyaA;
(d) a fragment corresponding to amino acids 1 to 317 of CyaA and a fragment corresponding to amino acids 318 to 400 of CyaA; and
(e) two fragments from eukaryotic adenylate cyclase in association with molecules, such as, G protein and forskolin.

The first and the second fragments can be a fragment T25 corresponding to amino acids 1 to 224 of *Bordetella pertussis* CyaA and a fragment T18 corresponding to amino acids 225 to 399 of *Bordetella pertussis* CyaA as described in WO 99/28746. In one embodiment, the first fragment is a mutated fragment of the catalytic domain of *Bordetella* adenylate cyclase (CyaA).

The modulating substance can be a natural activator, or a fragment thereof, of the enzyme. The natural activator can be the calmodulin (CaM), or a fragment thereof, and the first fragment can be mutated compared to the wild type enzyme. The fragment of calmodulin can be about 70 amino acids long, corresponding preferentially, to residues 77 to 148 of mammalian calmodulin, as described in WO 99/28746.

The present invention also concerns a method of selecting a molecule of interest which is capable of binding to target ligand wherein the interaction between the said molecule of interest and the said target ligand is detected with a signal amplification system of the invention, by means of generating a signal amplification and triggering transcriptional activation or repression, wherein the method of selecting the molecule of interest is performed in an *E. coli* strain selected from the group consisting of strain **BTH101** having C.N.C.M. Deposit Accession No. I-2309 and strain **DHM1** having C.N.C.M. Deposit Accession No. I-2310.

In a first embodiment, the signal amplification system according to the invention comprises a bacterial multi-hybrid system of at least two distinct fragments of an enzyme, whose enzymatic activity is restored by the interaction between the said molecule of interest and the said target ligand.

In a second embodiment, the signal amplification system according to the invention comprises bacterial multi-hybrid system of at least a first fragment of an enzyme and a modulating substance, whose activity is restored by the interaction between the said molecule of interest and the said target ligand.

The signal amplification system according to the invention comprises a bacterial multi-hybrid system as described in WO 99/28746.

In the method of selecting a molecule of interest of the invention, the interaction between the molecule of interest and the target ligand is detected, by means of signal amplification which triggers transcriptional activation, and is quantified by measuring the synthesis of the signaling molecule or the expression of the reporter gene.

The signal amplification corresponds to the production of a signaling molecule. This signaling molecule is any molecule capable of leading to a signaling cascade reaction. In one embodiment the signaling molecule corresponds to the synthesis of cAMP. In another embodiment the signaling molecule corresponds to the synthesis of cGMP.

The transcriptional activation leads to a reporter gene expression; the reporter gene is selected from the group consisting of gene coding for nutritional marker, such as lactose or maltose; gene conferring resistance to antibiotics such as ampicillin, chloramphenicol, kanamycin, or tetracyclin; a gene encoding for a toxin; a color marker, such as, fluorescent marker of the type of the Green Fluorescent Protein (GFP); a gene encoding phage receptor proteins or a fragment thereof, such as phage λ receptor, *lamB*, and any other gene giving a selectable phenotype.

In one embodiment, cAMP, upon binding to CAP, is able to activate the transcription of catabolic operons, allowing the bacteria to ferment carbohydrates, such as maltose or lactose, and to express the phage λ receptor, protein LamB, which could serve as a marker at the bacterial surface. This signal amplification system comprising this bacterial multi-hybrid system using **BTH101** or **DHM1** is able to reveal, for example, interactions between small peptides (GCN4 leucine zipper), bacterial (tyrosyl tRNA synthetase), or eukaryotic proteins (yeast Prp11/Prp21 complex).

Accordingly, specific reporter cassettes in which any gene of interest is fused to a cAMP/CAP dependent promoter can be designed. Thus, to facilitate the screening and the selection of complex libraries, the construction of such a simple selection system using an antibiotic resistance gene can be performed.

The reporter gene could be a toxin, not naturally present in bacteria, under the control of a cAMP/CAP-dependent promoter. This could be particularly useful to search for chemical compounds or mutations that abolish a given interaction between the target ligand and a molecule of interest. According to this construction, when association between the target ligand and a molecule of interest takes place, cAMP will be produced, the expression of the toxin gene will be switched on, and the cells will be killed. A substance capable of stimulating or inhibiting the interaction between the target ligand and the molecule of interest and that abolishes interaction will shut down toxin gene expression and will enable the cells to grow. An easy selection for substances that abolish interaction between the target ligand and the molecule of interest is resistance to phage λ. The phage receptor, the LamB protein, is the product of the *lamB* gene, which is part of the maltose regulon, therefore its expression requires cAMP. Substances that abolish interaction between the target ligand and the molecule of interest will abrogate cAMP synthesis and bacteria will become resistant to phage λ. As a result, the cells will grow.

Another selection scheme for compounds or mutations that abolish a given interaction could be designed by constructing a strain that harbors a selectable marker (i.e. a gene conferring resistance to antibiotics such as ampicillin chloramphenicol, kanamycin, tetracyclin, etc.) under the transcriptional control of a promoter that is repressed by cAMP/CAP. Such cAMP/CAP repressed promoter can be engineered by introducing a synthetic CAP binding site within the promotor region as shown by Morita et al. (Morita T, Shigesada K., Kimizuka F., Aiba H. (1988), "Regulatory effect of a synthetic CRP recognition sequence placed downstream of a promoter" Nucleic Acids Res. 16:7315-32).

The International Patent Applications n° WO 96/23898 (Thastrup O. et al.) and n° WO 97/11094 (Thastrup O. et al.), respectively, relating to a method of detecting biologically active substances as Green Fluorescent Protein (GFP), and the International Patent Application n° WO 97/07463 (Chalfie M. et al.) describing the uses of GFP, are herein incorporated by reference, and a novel variant of GFP.

The target ligand according to the invention is selected from the group consisting of protein, peptide, polypeptide, receptor, ligand, antigen, antibody, DNA binding protein, glycoprotein, lipoprotein and recombinant protein.

According to the method of selecting a molecule of interest, the molecule of interest is capable of interacting with the target ligand and possibly of binding to said target ligand. In a specific embodiment of the method of selecting a molecule of interest, the molecule of interest is a mutant molecule compared to the known wild type molecule, and said molecule of interest is tested for its capacity of interacting with the target ligand.

The present invention includes a molecule of interest identified by the method of selecting a molecule of interest according to the present invention. The present invention further includes a molecule of interest corresponding to a polynucleotide capable of expressing a molecule, which interacts with a fused target ligand coupled with an enzyme or a fragment thereof

In a preferred embodiment, the selection of the molecule of interest of the invention is performed in strain **DHM1.** In another preferred embodiment, the selection of the molecule of interest of the invention is performed in strain **BTH101.**

The present invention also concerns a kit for selecting molecule of interest, wherein said kit comprises: (a) a signal amplification system of the invention; (b) a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310; (c) a medium allowing the detection of the complementation selected from the group consisting of indicator or selective medium as minimal medium supplemented with lactose or maltose as unique carbon source, medium with antibiotics, medium to visualize fluorescence, luminescense, conventional medium, such as xGAL medium, and medium which allows the sorting by the presence of the phage receptor.

In another embodiment, the invention concerns a kit for selecting molecule of interest, wherein said kit comprises: (a) a signal amplification system of the invention wherein the molecule of interest is a mutant molecule compared to the known wild type molecule; (b) a signal amplification system of the invention wherein the molecule of interest is the known wild type molecule as the control; (c) a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310; (d) a medium allowing the detection of the complementation selected from the group consisting of indicator or selective medium as minimal medium supplemented with lactose or maltose as unique carbon source, medium with antibiotics, medium to visualize fluorescence, luminescence, conventional medium, such as xGAL medium, and medium which allows the sorting by the presence of the phage receptor for each signal amplification system; (e) means for detecting whether the signal amplification system with the mutant molecule is enhanced or inhibited with respect to the signal amplification system with wild type.

The present invention also concerns a method of screening for substance capable of stimulating or inhibiting the interaction between a target ligand and a molecule of interest wherein respectively the stimulating or the inhibiting activity is detected with a signal amplification system of the invention, by means of generating an amplification and respectively of triggering or of abolishing transcriptional activation wherein said signal amplification and said triggered or abolished transcriptional activation are compared with those obtained from an identical signal amplification system without any substance and wherein the method of screening for the substance is performed in an E. *coli* strain selected from the group consisting of strain **BTH101** having C.N.C.M. Deposit Accession No. I-2309 and strain **DHM1** having C.N.C.M. Deposit Accession No. I-2310.

In a preferred embodiment, the method of screening for substance capable of stimulating or inhibiting the interaction between a target ligand and a molecule of interest of the invention is characterized by the signal amplification system that comprises a bacterial multi-hybrid system of at least two distinct fragments of an enzyme, whose enzymatic activity is restored by the interaction between the said molecule of interest and the said target ligand. In another preferred embodiment, the method of screening for substance capable of stimulating or inhibiting the interaction between a target ligand and a molecule of interest of the invention is characterized by the signal amplification system that comprises a bacterial multi-hybrid system of at least a first fragment of an enzyme and a modulating substance, whose activity is restored by the interaction between the said molecule of interest and the said target ligand.

In the method of screening for substance capable of stimulating the interaction between a target ligand and a molecule of interest of the invention, the signal amplification corresponds to the production of a signaling molecule.

In the method of screening for substance capable of inhibiting the interaction between a target ligand and a molecule of interest of the invention, the signal amplification corresponding to the production of a signaling molecule is blocked or partially abolished.

In the method of screening for substance capable of stimulating the interaction between a target ligand and a molecule of interest of the invention, the transcriptional activation leads to a reporter gene expression.

In the method of screening for substance capable of inhibiting the interaction between a target ligand and a molecule of interest of the invention, the transcriptional activation leading to a reporter gene expression is blocked or partially abolished.

In the method of screening for substance capable of stimulating or inhibiting the interaction between a target ligand and a molecule of interest of the invention, the target ligand is selected from the group consisting of receptor, ligand, antigen, antibody, DNA binding protein, glycoprotein and lipoprotein, and the substance is selected from the group consisting of protein, glycoprotein, lipoprotein, ligand and any other drug having stimulating or inhibitory affinity.

In the method of screening for substance capable of stimulating or inhibiting the interaction between a target ligand and a molecule of interest of the invention, the signaling molecule corresponds to the synthesis of cAMP or to the synthesis of cGMP.

In the method of screening of the invention, the reporter gene is selected from the group consisting of gene coding for nutritional marker such as lactose, maltose; gene conferring resistance to antibiotics such as ampicillin, kanamycin or tetracyclin; gene encoding for toxin; color marker such as fluorescent marker of the type of the Green Fluorescent Protein (GFP); gene encoding for phage receptor proteins or fragment thereof such as phage λ receptor, *lamB* and any other gene giving a selectable phenotype.

In a preferred embodiment, in the method of screening of the invention, the molecule of interest is a mutant molecule compared to the known wild type molecule and said molecule of interest is tested for its capacity of interacting with the target ligand.

In a preferred embodiment, the screening for substance is performed in strain **DHM1.** In another preferred embodiment, the screening for substance is performed in strain **BTH101.**

The present invention also concerns a kit for screening for substance capable of stimulating or inhibiting the interaction between a target ligand and a molecule of interest, wherein said kit comprises (a) a signal amplification system of the invention with the substance capable of stimulating or inhibiting the interaction between a target ligand and a molecule of interest; (b) a signal amplification system of the invention without any substance as the control; (c) a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310; (d) a medium allowing the detection of the complementation selected from the group consisting of indicator plate or selective medium as minimal medium supplemented with lactose or maltose as unique carbon source, medium with antibiotics, medium to visualize fluorescence, conventional medium and medium which allows the sorting by the presence of the phage receptor and; (e) means for detecting whether the signal amplification system with the substance is enhanced or inhibited with respect to the signal amplification system without any substance.

Functional analysis of *B. pertussis* adenylate cyclase activity can be easily monitored in an *E. coli* strain deficient in endogenous adenylate cyclase. In *E. coli,* cAMP bound to the transcriptional activator, CAP (catabolite activator protein), is a pleiotropic regulator of the expression of various genes, including genes involved in the catabolism of carbohydrates, such as lactose or maltose (Ullmann, A. & Danchin, A. (1983) in *Advances in Cyclic Nucleotide Research* (Raven Press, New York), Vol. vol. 15, pp. 1-53). Hence, *E. coli* strains lacking cAMP are unable to ferment lactose or maltose. When the entire catalytic domain of CyaA (amino acids 1 to 399) is expressed in *E. coli cya* under the transcriptional and translational control of *lacZ* (plasmid pDIA5240), its calmodulin-independent residual activity is sufficient to complement an adenylate cyclase deficient strain and to restore its ability to ferment lactose or maltose (Ladant, D., Glaser, P. & Ullmann, A. (1992) *J. Biol. Chem.* **267**, 2244-2250). This can be scored either on indicator plates (i.e. LB-X-Gal or MacConkey media supplemented with maltose) or on selective media (minimal media supplemented with lactose or maltose as unique carbon source).

The present invention further relates to the a molecule of interest identified by the methods of the invention.

The present invention also concerns the substance capable of stimulating or inhibiting the interaction between a target ligand and a molecule of interest identified by the method of the invention.

The present invention also concerns the signal amplification system of the invention, wherein the bacterial multi-hybrid system contains: (a) a first chimeric polypeptide corresponding to a first fragment a of an enzyme; (b) a second chimeric polypeptide corresponding to a second fragment of an enzyme or a modulating substance capable of activating said enzyme; (c) a substance capable of stimulating or inhibiting the interaction between a target ligand and a molecule of interest, wherein the first fragment is fused to a molecule of interest and the second fragment or the modulating substance is fused to a target ligand and wherein the activity of the enzyme is restored by the interaction between the said molecule of interest and the said target ligand and wherein a signal amplification is generated; and a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310.

The present invention also concerns a DNA library containing a collection of vectors transformed in a bacterial multi-hybrid system, wherein each vector contains a polynucleotide coding for the molecule of interest fused to a polynucleotide encoding for a first or second fragment of an enzyme; the polynucleotide is selected from the group consisting of cDNA, RNA, genomic DNA, mitochondrial DNA. In a preferred embodiment, the DNA library of the invention is the H. *pylori* DNA library having C.N.C.M. Deposit Accession No. I-2367.

The invention further concerns the strain BTH101 having C.N.C.M. Deposit Accession No. I-2309 and the strain DHM1 having C.N.C.M. Deposit Accession No. I-2310.

In summary, this invention provides the construction and selection of two new *E. coli cya* strains that overcome the limitations of the DHP1 strain described in published PCT application WO 99/28746. The new strains, DHM1 and BTH101, exhibit a stable Cya⁻ phenotype: DHM1 has an internal deletion of about 200 bp within the *cya* gene, BTH101 harbors an uncharacterized mutation within the *cya* gene which is most likely a deletion, as no Cya+ revertant could be isolated from this strain.

Functional complementation between standard hybrid proteins appeared to be as efficient in BTH 10 as it is in DHP1. Although less efficient than in DHP1, functional complementation between the same set of standard hybrid proteins in DHM1 is still higher than in all other *E. coli cya*^{*-*} strains tested.

These two strains were used as recipient cells to perform a genome-wide library screen of *Helicobacter pylori* fragments or complete open reading frames, searching for polypeptides that can interact with defined "bait" proteins. These results show that, using these new strains, the bacterial two-hybrid screen can be applied for large scale analysis of protein-protein interactions.

As it appears from the teachings of the specification, the invention is not limited in scope to one or several of the above detailed embodiments; the present invention also embraces all the alternatives that can be performed by one skilled in the same technical field, without deviating from the subject or from the scope of the instant invention.

### REFERENCES

The following publications are cited herein. The entire disclosure of each publication is relied upon and incorporated by reference herein.
1. Glaser, P., Roy, A., A. Danchin, 1989, *J*. *Bacteriol*., 171: 5176-5178. Molecular characterization of two cya mutations, *cya-854* and *cyaR1*.
2. Karimova, G., J. Pidoux, A. Ullmann and D. Ladant, 1998, *Proc. Natl. Acad. Sci. U.S.A*. 95:5752-5756. A bacterial two-hybrid system based on a reconstituted signal transduction pathway.
3. Miller, J. H., 1972, Experiments in Molecular Genetics.
4. Wanner, B. L., 1986, *J*. *Mol. Biol.,* 191: 39-58. Novel regulatory mutants of the phosphate regulon in *Escherichia coli* K-12.
5. Sancar, A., Rupp, W.D., 1979. Physical map of the *recA* gene. *Proc. Natl. Acad. Sci. U.S.A.* 76:3144-3148.

## Claims

1. A signal amplification system comprising a bacterial multi-hybrid system of at least two chimeric polypeptides containing :
(a) a first chimeric polypeptide corresponding to a first fragment of an enzyme;
(b) a second chimeric polypeptide corresponding to a second fragment of an enzyme or a modulating substance capable of activating said enzyme,
wherein the first fragment is fused to a molecule of interest and the second fragment or the modulating substance is fused to a target ligand and wherein the activity of the enzyme is restored by the *in vivo* interaction between the said molecule of interest and the said target ligand and wherein a signal amplification is generated; and
wherein signal amplification is performed in an *E. coli* strain selected from the group consisting of strain **BTH101** having C.N.C.M. Deposit Accession No. I-2309 and strain **DHM1** having C.N.C.M. Deposit Accession No. I-2310.

2. The signal amplification system according to claim 1, wherein the enzyme is an enzyme selected from the group consisting of adenylate cyclase and guanylate cyclase from any origin.

3. The signal amplification system according to claim 2, wherein the enzyme is the catalytic domain of *Bordetella* adenylate cyclase (CyaA), located within the first 400 amino acid residues of the adenylate cyclase enzyme.

4. The signal amplification system according to claim 3, wherein the first and the second fragments are any combination of fragments from the same enzyme which *in vitro* functionally interact with the natural activator of said enzyme by restoring its activity.

5. The signal amplification system according to claim 4, wherein the first and the second fragments are selected from the group consisting of :
(a) a fragment T25 corresponding to amino acids 1 to 224 of CyaA and a fragment T18 corresponding to amino acids 225 to 399 of CyaA;
(b) a fragment corresponding to amino acids 1 to 224 of CyaA and a fragment corresponding to amino acids 224 to 384 of CyaA;
(c) a fragment corresponding to amino acids 1 to 137 of CyaA and a fragment corresponding to amino acids 138 to 400 of CyaA;
(d) a fragment corresponding to amino acids 1 to 317 of CyaA and a fragment corresponding to amino acids 318 to 400 of CyaA ;
(e) two fragments from eukaryotic adenylate cyclase in association with molecules such as G protein, forskolin.

6. The signal amplification system according to claim 4 or 5, wherein the first and the second fragments are a fragment T25 corresponding to amino acids 1 to 224 of *Bordetella pertussis* CyaA and a fragment T18 corresponding to amino acids 225 to 399 of *Bordetella pertussis* CyaA.

7. The signal amplification system according to any one of the claims 1 to 3, wherein the modulating substance is a natural activator, or a fragment thereof, of the enzyme.

8. The signal amplification system according to claim 7, wherein the natural activator is the calmodulin (CaM), or a fragment thereof, and said first fragment is mutated compared to the wild type enzyme.

9. The signal amplification system according to claim 8, wherein the first fragment is a mutated fragment of the catalytic domain of *Bordetella* adenylate cyclase (CyaA).

10. A method of selecting a molecule of interest which is capable of binding to target ligand wherein the interaction between the said molecule of interest and the said target ligand is detected with a signal amplification system according to any one of the claims 1 to 9, by means of generating a signal amplification and triggering transcriptional activation or repression, wherein the method of selecting the molecule of interest is performed in an *E. coli* strain selected from the group consisting of strain **BTH101** having C.N.C.M. Deposit Accession No. I-2309 and strain **DHM1** having C.N.C.M. Deposit Accession No. I-2310.

11. The method of selecting a molecule of interest according to claim 10, wherein the signal amplification corresponds to the production of a signaling molecule.

12. The method of selecting a molecule of interest according to claim 10,
wherein the transcriptional activation leads to a reporter gene expression.

13. The method of selecting a molecule of interest according to any one of claims 10 to 12, wherein the signal amplification system comprises a bacterial multi-hybrid system of at least two distinct fragments of an enzyme, whose enzymatic activity is restored by the interaction between the said molecule of interest and the said target ligand.

14. The method of selecting a molecule of interest according to any one of claims 10 to 12, wherein the signal amplification system comprises bacterial multi-hybrid system of at least a first fragment of an enzyme and a modulating substance, whose activity is restored by the interaction between the said molecule of interest and the said target ligand.

15. The method of selecting a molecule of interest according to any one of claims 10 to 14, wherein the target ligand is selected from the group consisting of protein, peptide, polypeptide, receptor, ligand, antigen, antibody, DNA binding protein, glycoprotein, lipoprotein and recombinant protein.

16. The method of selecting a molecule of interest according to any one of claims 10 to 15, wherein the molecule of interest is capable of interacting with the target ligand and/or capable of binding to said target ligand.

17. The method of selecting a molecule of interest according to any one of claims 10 to 16, wherein the interaction between the molecule of interest and the target ligand is detected, by means of signal amplification which triggers transcriptional activation, and is quantified by measuring the synthesis of the signaling molecule or the expression of the reporter gene.

18. The method of selecting a molecule of interest according to any one of claims 10 to 17, wherein the signaling molecule corresponds to the synthesis of cAMP.

19. The method of selecting a molecule of interest according to claim 13, wherein the signaling molecule corresponds to the synthesis of cGMP.

20. The method of selecting a molecule of interest according to claim 12, wherein the reporter gene is selected from the group consisting of gene coding for nutritional marker such as lactose, maltose; gene conferring resistance to antibiotics such as ampicillin, kanamycin or tetracyclin; gene encoding for toxin; color marker such as fluorescent marker of the type of the Green Fluorescent Protein (GFP); gene encoding for phage receptor proteins or fragment thereof such as phage λ receptor, *lamB* and any other gene giving a selectable phenotype.

21. The method of selecting a molecule of interest according to any one of claims 10 to 20, wherein the molecule of interest is a mutant molecule compared to the known wild type molecule and said molecule of interest is tested for its capacity of interacting with the target ligand.

22. The method of selecting a molecule of interest according to any one of claims 10 to 21, wherein the selection is performed in strain **DHM1.**

23. The method of selecting a molecule of interest according to any one of claims 10 to 21 wherein the selection is performed in strain **BTH101.**

24. A kit for selecting molecule of interest, wherein said kit comprises:
(a) a signal amplification system according to any one of claims 1 to 9;
(b) a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310;
(c) a medium allowing the detection of the complementation selected from the group consisting of indicator or selective medium as minimal medium supplemented with lactose or maltose as unique carbon source, medium with antibiotics, medium to visualize fluorescence, luminescense, conventional medium, such as xGAL medium, and medium which allows the sorting by the presence of the phage receptor.

25. A kit for selecting molecule of interest, wherein said kit comprises:
(a) a signal amplification system according to any one of claims 1 to 9 wherein the molecule of interest is a mutant molecule compared to the known wild type molecule;
(b) a signal amplification system according to any one of claims 1 to 9 wherein the molecule of interest is the known wild type molecule as the control;
(c) a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310;
(d) a medium allowing the detection of the complementation selected from the group consisting of indicator or selective medium as minimal medium supplemented with lactose or maltose as unique carbon source, medium with antibiotics, medium to visualize fluorescence, luminescence, conventional medium, such as xGAL medium, and medium which allows the sorting by the presence of the phage receptor for each signal amplification system;
(e) means for detecting whether the signal amplification system with the mutant molecule is enhanced or inhibited with respect to the signal amplification system with wild type.

26. A method of screening for substance capable of stimulating or inhibiting the interaction between a target ligand and a molecule of interest wherein respectively the stimulating or the inhibiting activity is detected with a signal amplification system according to any one of the claims 1 to 9, by means of generating an amplification and respectively of triggering or of abolishing transcriptional activation;
wherein said signal amplification and said triggered or abolished transcriptional activation are compared with those obtained from an identical signal amplification system without any substance; and
wherein the method of screening for the substance is performed in an *E*. *coli* strain selected from the group consisting of strain BTH101 having C.N.C.M. Deposit Accession No. I-2309 and strain **DHM1** having C.N.C.M. Deposit Accession No. I-2310.

27. The method of screening for substance capable of stimulating or inhibiting the interaction between a target ligand and a molecule of interest according to claim 26, wherein the signal amplification system comprises a bacterial multi-hybrid system of at least two distinct fragments of an enzyme, whose enzymatic activity is restored by the interaction between the said molecule of interest and the said target ligand.

28. The method of screening for substance capable of stimulating or inhibiting the interaction between a target ligand and a molecule of interest according to claim 26, wherein the signal amplification system comprises a bacterial multi-hybrid system of at least a first fragment of an enzyme and a modulating substance, whose activity is restored by the interaction between the said molecule of interest and the said target ligand.

29. The method of screening for substance capable of stimulating the interaction between a target ligand and a molecule of interest according to any one of claims 26 to 28, wherein the signal amplification corresponds to the production of a signaling molecule.

30. The method of screening for substance capable of inhibiting the interaction between a target ligand and a molecule of interest according to any one of claims 26 to 28, wherein the signal amplification corresponding to the production of a signaling molecule is blocked or partially abolished.

31. The method of screening for substance capable of stimulating the interaction between a target ligand and a molecule of interest according to any one of claims 26 to 29, wherein the transcriptional activation leads to a reporter gene expression.

32. The method of screening for substance capable of inhibiting the interaction between a target ligand and a molecule of interest according to any one of claims 26 to 28 and to claim 30, wherein the transcriptional activation leading to a reporter gene expression is blocked or partially abolished.

33. The method of screening for substance capable of stimulating or inhibiting the interaction between a target ligand and a molecule of interest according to any one of claims 26 to 32, wherein the target ligand is selected from the group consisting of receptor, ligand, antigen, antibody, DNA binding protein, glycoprotein and lipoprotein.

34. The method of screening for substance capable of stimulating or inhibiting the interaction between a target ligand and a molecule of interest according to any one of claims 26 to 33, wherein the substance is selected from the group consisting of proteins, glycoproteins, lipoproteins, and ligands.

35. The method of screening for substance capable of stimulating or inhibiting the interaction between a target ligand and a molecule of interest according to claim 29 or 30, wherein the signaling molecule corresponds to the synthesis of cAMP.

36. The method of screening for substance capable of stimulating or inhibiting the interaction between a target ligand and a molecule of interest according to claim 29 or 30, wherein the signaling molecule corresponds to the synthesis ofcGMP.

37. The method of screening for substance capable of stimulating or inhibiting the interaction between a target ligand and a molecule of interest according to claim 31 or 32, wherein the reporter gene is selected from the group consisting of gene coding for nutritional marker such as lactose, maltose; gene conferring resistance to antibiotics such as ampicillin, kanamycin or tetracyclin; gene encoding for toxin; color marker such as fluorescent marker of the type of the Green Fluorescent Protein (GFP); gene encoding for phage receptor proteins or fragment thereof such as phage λ receptor, *lamB* and any other gene giving a selectable phenotype.

38. The method of screening for substance capable of stimulating or inhibiting the interaction between a target ligand and a molecule of interest according to any one of claims 26 to 37, wherein the molecule of interest is a mutant molecule compared to the known wild type molecule and said molecule of interest is tested for its .capacity of interacting with the target ligand.

39. The method of screening for substance capable of stimulating or inhibiting the interaction between a target ligand and a molecule of interest according to any one of claims 26 to 37, wherein the screening is performed in strain **DHM1.**

40. The method of screening for a substance capable of stimulating or inhibiting the interaction between a target ligand and a molecule of interest according to any one of claims 26 to 38, wherein the screening is performed in strain **BTH101.**

41. A kit for screening for substance capable of stimulating or inhibiting the interaction between a target ligand and a molecule of interest, wherein said kit comprises:
(a) a signal amplification system according to any one of claims 1 to 9 with the substance capable of stimulating or inhibiting the interaction between a target ligand and a molecule of interest;
(b) a signal amplification system according to any one of claims 1 to 9 without any substance as the control;
(c) a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310;
(d) a medium allowing the detection of the complementation selected from the group consisting of indicator plate or selective medium as minimal medium supplemented with lactose or maltose as unique carbon source, medium with antibiotics, medium to visualize fluorescence, conventional medium and medium which allows the sorting by the presence of the phage receptor and;
(e) means for detecting whether the signal amplification system with the substance is enhanced or inhibited with respect to the signal amplification system without any substance.

42. The signal amplification system according to any one of the claims 1 to 9, wherein the bacterial multi-hybrid system contains:
(a) a first chimeric polypeptide corresponding to a first fragment a of an enzyme;
(b) a second chimeric polypeptide corresponding to a second fragment of an enzyme or a modulating substance capable of activating said enzyme;
(c) a substance capable of stimulating or inhibiting the interaction between a target ligand and a molecule of interest,' wherein the first fragment is fused to a molecule of interest and the second fragment or the modulating substance is fused to a target ligand and wherein the activity of the enzyme is restored by the interaction between the said molecule of interest and the said target ligand and wherein a signal amplification is generated; and
a bacterial strain deficient in endogenous adenylate cyclase selected from the group consisting of strain **BTH101** having C.N.C.M. Deposit Accession No. I-2309 and strain **DHM1** having C.N.C.M. Deposit Accession No. I-2310.

43. Strain **BTH101** having C.N.C.M. Deposit Accession No. I-2309.

44. Strain **DHM1** having C.N.C.M. Deposit Accession No. I-2310.

## Patentansprüche

1. Signalamplifizierungssystem, umfassend ein bakterielles Multi-Hybrid-System aus wenigstens zwei chimären Polypeptiden, enthaltend:
(a) ein erstes chimäres Polypeptid, welches einem ersten Fragment eines Enzyms entspricht;
(b) ein zweites chimäres Polypeptid, welches einem zweiten Fragment eines Enzyms oder einer modulierenden Substanz, welche in der Lage ist, das Enzym zu aktivieren, entspricht,
wobei das erste Fragment mit einem Molekül von Interesse fusioniert ist und das zweite Fragment oder die modulierende Substanz mit einem Ziel-Liganden fusioniert ist und wobei die Aktivität des Enzyms durch die in vivo-Wechselwirkung zwischen dem Molekül von Interesse und dem Ziel-Liganden wiederhergestellt wird und
wobei eine Signalamplifizierung erzeugt wird; und
wobei die Signalamplifizierung in einem E. coli-Stamm, ausgewählt aus der Gruppe bestehend aus dem Stamm **BTH101** mit der "C.N.C.M. Deposit Accession No." (C.N.C.M.-Hinterlegungs-Aufnahme-Nr.) 1-2309 und dem Stamm **DHM1** mit der "C.N.C.M. Deposit Accession No." I-2310, ausgeführt wird.

2. Signalamplifizierungssystem nach Anspruch 1, wobei das Enzym ein Enzym ist, welches aus der Gruppe bestehend aus Adenylatcyclase und Guanylatcyclase von beliebiger Herkunft ausgewählt wird.

3. Signalamplifizierungssystem nach Anspruch 2, wobei das Enzym die katalytische Domäne der *Bordetella*-Adenylatcyclase (CyaA) ist, welche sich innerhalb der ersten 400 Aminosäurereste des Adenylatcyclaseenzyms befindet.

4. Signalamplifizierungssystem nach Anspruch 3, wobei das erste und das zweite Fragment eine beliebige Kombination von Fragmenten aus dem gleichen Enzym, welche *in vitro* in funktionaler Weise mit dem natürlichen Aktivator des Enzyms wechselwirken, indem dessen Aktivität wiederhergestellt wird, sind.

5. Signalamplifizierungssystem nach Anspruch 4, wobei das erste und das zweite Fragment ausgewählt werden aus der Gruppe bestehend aus:
(a) einem Fragment T25, welches den Aminosäuren 1 bis 224 von CyaA entspricht, und einem Fragment T18, welches den Aminosäuren 225 bis 399 von CyaA entspricht;
(b) einem Fragment, welches den Aminosäuren 1 bis 224 von CyaA entspricht, und einem Fragment, welches den Aminosäuren 224 bis 384 von CyaA entspricht;
(c) einem Fragment, welches den Aminosäuren 1 bis 137 von CyaA entspricht, und einem Fragment, welches den Aminosäuren 138 bis 400 von CyaA entspricht;
(d) einem Fragment, welches den Aminosäuren 1 bis 317 von CyaA entspricht, und einem Fragment, welches den Aminosäuren 318 bis 400 von CyaA entspricht;
(e) zwei Fragmenten aus einer eukaryotischen Adenylatcyclase in Kombination mit Molekülen, wie G-Protein, Forskolin.

6. Signalamplifizierungssystem nach Anspruch 4 oder 5, wobei das erste und das zweite Fragment ein Fragment T25, welches den Aminosäuren 1 bis 224 von *Bordetella pertussis*-CyaA entspricht, und ein Fragment T18, welches den Aminosäuren 225 bis 399 von *Bordetella pertussis*-CyaA entspricht, sind.

7. Signalamplifizierungssystem nach einem der Ansprüche 1 bis 3, wobei die modulierende Substanz ein natürlicher Aktivator des Enzyms oder ein Fragment dieses natürlichen Aktivators ist.

8. Signalamplifizierungssystem nach Anspruch 7, wobei der natürliche Aktivator Calmodulin (CaM) oder ein Fragment davon ist und das erste Fragment verglichen mit dem Wildtyp-Enzym mutiert ist.

9. Signalamplifizierungssystem nach Anspruch 8, wobei das erste Fragment ein mutiertes Fragment der katalytischen Domäne von *Bordetella*-Adenylatcyclase (CyaA) ist.

10. Verfahren zum Selektieren eines Moleküls von Interesse, welches in der Lage ist, an einen Ziel-Liganden zu binden, wobei die Wechselwirkung zwischen dem Molekül von Interesse und dem Ziel-Liganden mit einem Signalamplifizierungssystem nach einem der Ansprüche 1 bis 9 mittels der Erzeugung einer Signalamplifizierung und Triggern von Transkriptionsaktivierung oder-repression detektiert wird, wobei das Verfahren zum Selektieren des Moleküls von Interesse in einem *E. coli*-Stamm, ausgewählt aus der Gruppe bestehend aus dem Stamm **BTH101** mit der "C.N.C.M. Deposit Accession No." I-2309 und dem Stamm **DHM1** mit der "C.N.C.M. Deposit Accession No." I-2310, ausgeführt wird.

11. Verfahren zum Selektieren eines Moleküls von Interesse nach Anspruch 10, wobei die Signalamplifizierung der Produktion eines Signalstoffs entspricht.

12. Verfahren zum Selektieren eines Moleküls von Interesse nach Anspruch 10, wobei die Transkriptionsaktivierung zu der Expression eines Reportergens führt.

13. Verfahren zum Selektieren eines Moleküls von Interesse nach einem der Ansprüche 10 bis 12, wobei das Signalamplifizierungssystem ein bakterielles Multi-Hybrid-System aus wenigstens zwei unterschiedlichen Fragmenten eines Enzyms, dessen enzymatische Aktivität durch die Wechselwirkung zwischen dem Molekül von Interesse und dem Ziel-Liganden wiederhergestellt wird, umfasst.

14. Verfahren zum Selektieren eines Moleküls von Interesse nach einem der Ansprüche 10 bis 12, wobei das Signalamplifizierungssystem ein bakterielles Multi-Hybrid-System aus wenigstens einem ersten Fragment eines Enzyms und einer modulierenden Substanz, deren Aktivität durch die Wechselwirkung zwischen dem Molekül von Interesse und dem Ziel-Liganden wiederhergestellt wird, umfasst.

15. Verfahren zum Selektieren eines Moleküls von Interesse nach einem der Ansprüche 10 bis 14, wobei der Ziel-Ligand aus der Gruppe bestehend aus Protein, Peptid, Polypeptid, Rezeptor, Ligand, Antigen, Antikörper, DNA-bindendem Protein, Glycoprotein, Lipoprotein und rekombinantem Protein ausgewählt wird.

16. Verfahren zum Selektieren eines Moleküls von Interesse nach einem der Ansprüche 10 bis 15, wobei das Molekül von Interesse in der Lage ist, mit dem Ziel-Liganden zu wechselwirken, und/oder in der Lage ist, an den Ziel-Liganden zu binden.

17. Verfahren zum Selektieren eines Moleküls von Interesse nach einem der Ansprüche 10 bis 16, wobei die Wechselwirkung zwischen dem Molekül von Interesse und dem Ziel-Liganden mittels einer Signalamplifizierung, welche Transkriptionsaktivierung triggert, nachgewiesen und durch Messen der Synthese des Signalstoffs oder der Expression des Reportergens quantifiziert wird.

18. Verfahren zum Selektieren eines Moleküls von Interesse nach einem der Ansprüche 10 bis 17, wobei der Signalstoff der Synthese von cAMP entspricht.

19. Verfahren zum Selektieren eines Moleküls von Interesse nach Anspruch 13, wobei der Signalstoff der Synthese von cGMP entspricht.

20. Verfahren zum Selektieren eines Moleküls von Interesse nach Anspruch 12, wobei das Reportergen ausgewählt wird aus der Gruppe bestehend aus einem für einen Ernährungsmarker, wie Lactose, Maltose, kodierenden Gen; einem Resistenz gegen Antibiotika, wie Ampicillin, Kanamycin oder Tetracyclin, verleihenden Gen; einem ein Toxin kodierenden Gen; einem Farbmarker, wie einem fluoreszierenden Marker vom Typ des "Green Fluorescent Protein" (GFP); einem Phagenrezeptorproteine oder ein Fragment davon, wie den Phage λ-Rezeptor, kodierenden Gen, *lamB* und einem jeglichen anderen Gen, welches einen selektierbaren Phänotyp ergibt.

21. Verfahren zum Selektieren eines Moleküls von Interesse nach einem der Ansprüche 10 bis 20, wobei das Molekül von Interesse ein verglichen mit dem bekannten Wildtyp-Molekül mutiertes Molekül ist und das Molekül von Interesse auf dessen Vermögen zur Wechselwirkung mit dem Ziel-Liganden hin untersucht wird.

22. Verfahren zum Selektieren eines Moleküls von Interesse nach einem der Ansprüche 10 bis 21, wobei die Selektion in dem Stamm **DHM1** ausgeführt wird.

23. Verfahren zum Selektieren eines Moleküls von Interesse nach einem der Ansprüche 10 bis 21, wobei die Selektion in dem Stamm **BTH101** ausgeführt wird.

24. Kit zum Selektieren eines Moleküls von Interesse, wobei der Kit umfasst:
(a) ein Signalamplifizierungssystem nach einem der Ansprüche 1 bis 9;
(b) einen Bakterienstamm, welcher einen Mangel an endogener Adenylatcyclase aufweist, ausgewählt aus der Gruppe bestehend aus dem Stamm BTH101 mit der "C.N.C.M. Deposit Accession No." 1-2309 und dem Stamm **DHM1** mit der "C.N.C.M. Deposit Accession No." I-2310;
(c) ein Medium, welches den Nachweis der Komplementation erlaubt, ausgewählt aus der Gruppe bestehend aus einem Indikatoroder selektiven Medium, wie mit Lactose oder Maltose als einziger Kohlenstoffquelle ergänztem Minimalmedium, Medium mit Antibiotika, Medium, um Fluoreszenz, Lumineszenz sichtbar zu machen, herkömmlichem Medium, wie xGAL-Medium, und Medium, welches das Sortieren aufgrund der Anwesenheit des Phagenrezeptors erlaubt.

25. Kit zum Selektieren eines Moleküls von Interesse, wobei der Kit umfasst:
(a) ein Signalamplifizierungssystem nach einem der Ansprüche 1 bis 9, wobei das Molekül von Interesse ein verglichen mit dem bekannten Wildtyp-Molekül mutiertes Molekül ist;
(b) ein Signalamplifizierungssystem nach einem der Ansprüche 1 bis 9, wobei das Molekül von Interesse das bekannte Wildtyp-Molekül ist, als Kontrolle;
(c) einen Bakterienstamm, welcher einen Mangel an endogener Adenylatcyclase aufweist, ausgewählt aus der Gruppe bestehend aus dem Stamm **BTH101** mit der "C.N.C.M. Deposit Accession No." I-2309 und dem Stamm **DHM1** mit der "C.N.C.M. Deposit Accession No." I-2310;
(d) ein Medium, welches für jedes Signalamplifizierungssystem den Nachweis der Komplementation erlaubt, ausgewählt aus der Gruppe, bestehend aus einem Indikator- oder selektiven Medium, wie mit Lactose oder Maltose als einziger Kohlenstoffquelle ergänztem Minimalmediüm, Medium mit Antibiotika, Medium, um Fluoreszenz, Lumineszenz sichtbar zu machen, herkömmlichem Medium, wie xGAL-Medium, und Medium, welches das Sortieren aufgrund der Anwesenheit des Phagenrezeptors erlaubt;
(e) Mittel zum Nachweisen, ob das Signalamplifizierungssystem mit dem mutierten Molekül bezogen auf das Signalamplifizierungssystem mit dem Wildtyp verstärkt oder gehemmt ist.

26. Verfahren zum Screenen auf eine Substanz, welche in der Lage ist, die Wechselwirkung zwischen einem Ziel-Liganden und einem Molekül von Interesse zu stimulieren oder zu hemmen, wobei die stimulierende bzw. hemmende Aktivität mit einem Signalamplifizierungssystem nach einem der Ansprüche 1 bis 9 nachgewiesen wird mittels der Erzeugung einer Amplifizierung und des Triggerns bzw. des Vernichtens von Transkriptionsaktivierung;
wobei die Signalamplifizierung und die getriggerte oder vernichtete Transkriptionsaktivierung mit jenen, die ausgehend von einem identischen Signalamplifizierungssystem ohne jegliche Substanz erhalten werden, verglichen werden; und
wobei das Verfahren zum Screenen auf die Substanz in einem *E. coli*-Stamm, ausgewählt aus der Gruppe bestehend aus dem Stamm **BTH101** mit der "C.N.C.M. Deposit Accession No." I-2309 und dem Stamm **DHM1** mit der "C.N.C.M. Deposit Accession No." I-2310, ausgeführt wird.

27. Verfahren zum Screenen auf eine Substanz, welche in der Lage ist, die Wechselwirkung zwischen einem Ziel-Liganden und einem Molekül von Interesse zu stimulieren oder zu hemmen, nach Anspruch 26, wobei das Signalamplifizierungssystem ein bakterielles Multi-Hybrid-System aus wenigstens zwei unterschiedlichen Fragmenten eines Enzyms, dessen enzymatische Aktivität durch die Wechselwirkung zwischen dem Molekül von Interesse und dem Ziel-Liganden wiederhergestellt wird, umfasst.

28. Verfahren zum Screenen auf eine Substanz, welche in der Lage ist, die Wechselwirkung zwischen einem Ziel-Liganden und einem Molekül von Interesse zu stimulieren oder zu hemmen, nach Anspruch 26, wobei das Signalamplifizierungssystem ein bakterielles Multi-Hybrid-System aus wenigstens einem ersten Fragment eines Enzyms und einer modulierenden Substanz, deren Aktivität durch die Wechselwirkung zwischen dem Molekül von Interesse und dem Ziel-Liganden wiederhergestellt wird, umfasst.

29. Verfahren zum Screenen auf eine Substanz, welche in der Lage ist, die Wechselwirkung zwischen einem Ziel-Liganden und einem Molekül von Interesse zu stimulieren, nach einem der Ansprüche 26 bis 28, wobei die Signalamplifizierung der Produktion eines Signalstoffs entspricht.

30. Verfahren zum Screenen auf eine Substanz, welche in der Lage ist, die Wechselwirkung zwischen einem Ziel-Liganden und einem Molekül von Interesse zu hemmen, nach einem der Ansprüche 26 bis 28, wobei die Signalamplifizierung, welche der Produktion eines Signalstoffs entspricht, blockiert oder teilweise vernichtet wird.

31. Verfahren zum Screenen auf eine Substanz, welche in der Lage ist, die Wechselwirkung zwischen einem Ziel-Liganden und einem Molekül von Interesse zu stimulieren, nach einem der Ansprüche 26 bis 29, wobei die Transkriptionsaktivierung zu der Expression eines Reportergens führt.

32. Verfahren zum Screenen auf eine Substanz, welche in der Lage ist, die Wechselwirkung zwischen einem Ziel-Liganden und einem Molekül von Interesse zu hemmen, nach einem der Ansprüche 26 bis 28 und nach Anspruch 30, wobei die Transkriptionsaktivierung, welche zu der Expression eines Reportergens führt, blockiert oder teilweise vernichtet wird.

33. Verfahren zum Screenen auf eine Substanz, welche in der Lage ist, die Wechselwirkung zwischen einem Ziel-Liganden und einem Molekül von Interesse zu stimulieren oder zu hemmen, nach einem der Ansprüche 26 bis 32, wobei der Ziel-Ligand aus der Gruppe bestehend aus Rezeptor, Ligand, Antigen, Antikörper, DNA-bindendem Protein, Glycoprotein und Lipoprotein ausgewählt wird.

34. Verfahren zum Screenen auf eine Substanz, welche in der Lage ist, die Wechselwirkung zwischen einem Ziel-Liganden und einem Molekül von Interesse zu stimulieren oder zu hemmen, nach einem der Ansprüche 26 bis 33, wobei die Substanz aus der Gruppe bestehend aus Proteinen, Glycoproteinen, Lipoproteinen und Liganden ausgewählt wird.

35. Verfahren zum Screenen auf eine Substanz, welche in der Lage ist, die Wechselwirkung zwischen einem Ziel-Liganden und einem Molekül von Interesse zu stimulieren oder zu hemmen, nach Anspruch 29 oder 30, wobei der Signalstoff der Synthese von cAMP entspricht.

36. Verfahren zum Screenen auf eine Substanz, welche in der Lage ist, die Wechselwirkung zwischen einem Ziel-Liganden und einem Molekül von Interesse zu stimulieren oder zu hemmen, nach Anspruch 29 oder 30, wobei der Signalstoff der Synthese von cGMP entspricht.

37. Verfahren zum Screenen auf eine Substanz, welche in der Lage ist, die Wechselwirkung zwischen einem Ziel-Liganden und einem Molekül von Interesse zu stimulieren oder zu hemmen, nach Anspruch 31 oder 32, wobei das Reportergen ausgewählt wird aus der Gruppe bestehend aus einem für einen Ernährungsmarker, wie Lactose, Maltose, kodierenden Gen; einem Resistenz gegen Antibiotika, wie Ampicillin, Kanamycin oder Tetracyclin, verleihenden Gen; einem ein Toxin kodierenden Gen; einem Farbmarker, wie einem fluoreszierenden Marker vom Typ des "Green Fluorescent Protein" (GFP); einem Phagenrezeptorproteine oder ein Fragment davon, wie den Phage λ-Rezeptor, kodierenden Gen, *lamB* und einem jeglichen anderen Gen, welches einen selektierbaren Phänotyp ergibt.

38. Verfahren zum Screenen auf eine Substanz, welche in der Lage ist, die Wechselwirkung zwischen einem Ziel-Liganden und einem Molekül von Interesse zu stimulieren oder zu hemmen, nach einem der Ansprüche 26 bis 37, wobei das Molekül von Interesse ein verglichen mit dem bekannten Wildtyp-Molekül mutiertes Molekül ist und das Molekül von Interesse auf dessen Vermögen zur Wechselwirkung mit dem Ziel-Liganden hin untersucht wird.

39. Verfahren zum Screenen auf eine Substanz, welche in der Lage ist, die Wechselwirkung zwischen einem Ziel-Liganden und einem Molekül von Interesse zu stimulieren oder zu hemmen, nach einem der Ansprüche 26 bis 37, wobei das Screenen in dem Stamm **DHM1** ausgeführt wird.

40. Verfahren zum Screenen auf eine Substanz, welche in der Lage ist, die Wechselwirkung zwischen einem Ziel-Liganden und einem Molekül von Interesse zu stimulieren oder zu hemmen, nach einem der Ansprüche 26 bis 38, wobei das Screenen in dem Stamm BTH101 ausgeführt wird.

41. Kit zum Screenen auf eine Substanz, welche in der Lage ist, die Wechselwirkung zwischen einem Ziel-Liganden und einem Molekül von Interesse zu stimulieren oder zu hemmen, wobei der Kit umfasst:
(a) ein Signalamplifizierungssystem nach einem der Ansprüche 1 bis 9 mit der Substanz, welche in der Lage ist, die Wechselwirkung zwischen einem Ziel-Liganden und einem Molekül von Interesse zu stimulieren oder zu hemmen;
(b) ein Signalamplifizierungssystem nach einem der Ansprüche 1 bis 9 ohne jegliche Substanz als Kontrolle;
(c) einen Bakterienstamm, welcher einen Mangel an endogener Adenylatcyclase aufweist, ausgewählt aus der Gruppe bestehend aus dem Stamm **BTH101** mit der "C.N.C.M. Deposit Accession No." I-2309 und dem Stamm **DHM1** mit der "C.N.C.M. Deposit Accession No." I-2310;
(d) ein Medium, welches den Nachweis der Komplementation erlaubt, ausgewählt aus der Gruppe, bestehend aus einer Indikatorplatte oder einem selektiven Medium, wie mit Lactose oder Maltose als einziger Kohlenstoffquelle ergänztem Minimalmedium, Medium mit Antibiotika, Medium, um Fluoreszenz sichtbar zu machen, herkömmlichem Medium und Medium, welches das Sortieren aufgrund der Anwesenheit des Phagenrezeptors erlaubt;
(e) Mittel zum Nachweisen, ob das Signalamplifizierungssystem mit der Substanz bezogen auf das Signalamplifizierungssystem ohne jegliche Substanz verstärkt oder gehemmt ist.

42. Signalamplifizierungssystem nach einem der Ansprüche 1 bis 9, wobei das bakterielle Multi-Hybrid-System enthält:
(a) ein erstes chimäres Polypeptid, welches einem ersten Fragment eines Enzyms entspricht;
(b) ein zweites chimäres Polypeptid, welches einem zweiten Fragment eines Enzyms oder einer modulierenden Substanz, welche in der Lage ist, das Enzym zu aktivieren, entspricht;
(c) eine Substanz, welche in der Lage ist, die Wechselwirkung zwischen einem Ziel-Liganden und einem Molekül von Interesse zu stimulieren oder zu hemmen, wobei das erste Fragment mit einem Molekül von Interesse fusioniert ist und das zweite Fragment oder die modulierende Substanz mit einem Ziel-Liganden fusioniert ist und wobei die Aktivität des Enzyms durch die Wechselwirkung zwischen dem Molekül von Interesse und dem Ziel-Liganden wiederhergestellt wird und wobei eine Signalamplifizierung erzeugt wird; und
einen Bakterienstamm, welcher einen Mangel an endogener Adenylatcyclase aufweist, ausgewählt aus der Gruppe bestehend aus dem Stamm **BTH101** mit der "C.N.C.M. Deposit Accession No." I-2309 und dem Stamm **DHM1** mit der "C.N.C.M. Deposit Accession No." I-2310.

43. Stamm **BTH101** mit der "C.N.C.M. Deposit Accession No." I-2309.

44. Stamm **DHM1** mit der "C.N.C.M. Deposit Accession No." I-2310.

## Revendications

1. Système d'amplification de signal comprenant un système bactérien multi-hybride d'au moins deux polypeptides chimères contenant :
(a) un premier polypeptide chimère correspondant à un premier fragment d'une enzyme ;
(b) un second polypeptide chimère correspondant à un second fragment d'une enzyme ou à une substance modulante capable d'activer ladite enzyme,
dans lequel le premier fragment est fusionné à une molécule présentant un intérêt et le second fragment ou la molécule modulante est fusionné à un ligand cible, l'activité de l'enzyme étant restaurée par l'interaction *in vivo* entre ladite molécule présentant un intérêt et ledit ligand cible et une amplification de signal étant générée ; et
où l'amplification du signal est réalisée dans une souche d'E. *coli* choisie dans le groupe constitué par la souche **BTH101** dont le numéro d'enregistrement de dépôt à la Collection Nationale de Cultures de Microorganismes (C.N.C.M.) est I-2309 et la souche **DHM1** dont le numéro d'enregistrement de dépôt à la C.N.C.M. est I-2310.

2. Système d'amplification de signal selon la revendication 1, dans lequel l'enzyme est une enzyme choisie dans le groupe constitué par une adénylate cyclase et une guanylate cyclase d'origine quelconque.

3. Système d'amplification de signal selon la revendication 2, dans lequel l'enzyme est le domaine catalytique de l'adénylate cyclase de *Bordetella* (CyaA), localisé dans les 400 premiers restes d'aminoacides de l'enzyme adénylate cyclase.

4. Système d'amplification de signal selon la revendication 3, dans lequel le premier et le second fragments sont constitués par une combinaison quelconque de fragments de la même enzyme qui interagit fonctionnellement *in vitro* avec l'activateur naturel de la dite enzyme en restaurant l'activité de celle-ci.

5. Système d'amplification de signal selon la revendication 4, dans lequel le premier et le second fragments sont choisis dans le groupe constitué par :
(a) un fragment T25 correspondant aux aminoacides 1 à 224 de CyaA et un fragment T18 correspondant aux aminoacides 225 à 399 de CyaA ;
(b) un fragment correspondant aux aminoacides 1 à 224 de CyaA et un fragment correspondant aux aminoacides 224 à 3 84 de CyaA ;
(c) un fragment correspondant aux aminoacides 1 à 137 de CyaA et un fragment correspondant aux aminoacides 13 8 à 400 de CyaA ;
(d) un fragment correspondant aux aminoacides 1 à 317 de CyaA et un fragment correspondant aux aminoacides 318 à 400 de CyaA ;
(e) deux fragments d'une adénylate cyclase d'eucaryote en association avec des molécules telles que la protéine G, la forskoline.

6. Système d'amplification de signal selon la revendication 4 ou 5, dans lequel le premier et le second fragments sont un fragment T25 correspondant aux aminoacides 1 à 224 de CyaA de *Bordetella pertussis* et un fragment T18 correspondant aux aminoacides 225 à 399 de CyaA de *Bordetella pertussis.*

7. Système d'amplification de signal selon l'une quelconque des revendications 1 à 3, dans lequel la substance modulante est un activateur naturel de l'enzyme, ou un fragment d'un tel activateur.

8. Système d'amplification de signal selon la revendication 7, dans lequel l'activateur naturel est la calmoduline (CaM), ou un fragment de celle-ci, et ledit premier fragment est muté par rapport à l'enzyme du type sauvage.

9. Système d'amplification de signal selon la revendication 8, dans lequel le premier fragment est un fragment muté du domaine catalytique de l'adénylate cyclase de *Bordetella* (CyaA).

10. Méthode de sélection d'une molécule présentant un intérêt, capable de se lier à un ligand cible, l'interaction entre ladite molécule présentant un intérêt et ledit ligand cible étant détectée par un système d'amplification de signal selon l'une quelconque des revendications 1 à 9, au moyen de la génération d'une amplification de signal et du déclenchement de l'activation ou de la répression de la transcription, dans laquelle la méthode de sélection de la molécule présentant un intérêt est réalisée dans une souche d'*E. coli* choisie dans le groupe constitué par la souche **BTH101** dont le numéro d'enregistrement de dépôt à la C.N.C.M. est I-2309 et la souche **DHM1** dont le numéro d'enregistrement de dépôt à la C.N.C.M. est I-2310.

11. Méthode de sélection d'une molécule présentant un intérêt selon la revendication 10, dans laquelle l'amplification de signal correspond à la production d'une molécule signalisante.

12. Méthode de sélection d'une molécule présentant un intérêt selon la revendication 10, dans laquelle l'activation de la transcription conduit à l'expression d'un gène reporteur.

13. Méthode de sélection d'une molécule présentant un intérêt selon l'une quelconque des revendications 10 à 12, dans laquelle le système d'amplification de signal comprend un système bactérien multi-hybride d'au moins deux fragments distincts d'une enzyme, dont l'activité enzymatique est restaurée par l'interaction entre ladite molécule présentant un intérêt et ledit ligand cible.

14. Méthode de sélection d'une molécule présentant un intérêt selon l'une quelconque des revendications 10 à 12, dans laquelle le système d'amplification de signal comprend un système bactérien multi-hybride d'au moins un premier fragment d'une enzyme et une substance modulante, dont l'activité est restaurée par l'interaction entre ladite molécule présentant un intérêt et ledit ligand cible.

15. Méthode de sélection d'une molécule présentant un intérêt selon l'une quelconque des revendications 10 à 14, dans laquelle le ligand cible est choisi dans le groupe constitué par une protéine, un peptide, un polypeptide, un récepteur, un ligand, un antigène, un anticorps, une protéine se liant à l'ADN, une glycoprotéine, une lipoprotéine et une protéine recombinée.

16. Méthode de sélection d'une molécule présentant un intérêt selon l'une quelconque des revendications 10 à 15, dans laquelle la molécule présentant un intérêt est capable d'interagir avec le ligand cible et/ou est capable de se lier audit ligand cible.

17. Méthode de sélection d'une molécule présentant un intérêt selon l'une quelconque des revendications 10 à 16, dans laquelle l'interaction entre la molécule présentant un intérêt et le ligand cible est détectée au moyen d'une amplification de signal qui déclenche l'activation de la transcription, et est quantifiée par la mesure de la synthèse de la molécule signalisante ou de l'expression du gène reporteur.

18. Méthode de sélection d'une molécule présentant un intérêt selon l'une quelconque des revendications 10 à 17, dans laquelle la molécule signalisante correspond à la synthèse d'AMPc.

19. Méthode de sélection d'une molécule présentant un intérêt selon la revendication 13, dans laquelle la molécule signalisante correspond à la synthèse de GMPc.

20. Méthode de sélection d'une molécule présentant un intérêt selon la revendication 12, dans laquelle le gène reporteur est choisi dans le groupe constitué par un gène codant pour un marqueur nutritionnel comme le lactose, le maltose ; un gène conférant une résistance à des antibiotiques comme l'ampicilline, la kanamycine ou la tétracycline ; un gène codant pour une toxine ; un marqueur coloré comme un marqueur fluorescent du type de la protéine fluorescente verte (GFP); un gène codant pour des protéines réceptrices de phage ou un fragment de celles-ci comme le récepteur de phage λ, *lamB* et tout autre gène donnant un phénotype pouvant donner lieu à une sélection.

21. Méthode de sélection d'une molécule présentant un intérêt selon l'une quelconque des revendications 10 à 20, dans laquelle la molécule présentant un intérêt est une molécule mutante par rapport à la molécule connue du type sauvage et ladite molécule présentant un intérêt est testée pour déterminer sa capacité à interagir avec le ligand cible.

22. Méthode de sélection d'une molécule présentant un intérêt selon l'une quelconque des revendications 10 à 21, dans laquelle la sélection est effectuée dans la souche **DHM1.**

23. Méthode de sélection d'une molécule présentant un intérêt selon l'une quelconque des revendications 10 à 21, dans laquelle la sélection est effectuée dans la souche BTH101.

24. Kit de sélection d'une molécule présentant un intérêt, lédit kit comprenant :
(a) un système d'amplification de signal selon l'une quelconque des revendications 1 à 9 ;
(b) une souche bactérienne carencée en adénylate cyclase endogène choisie dans le groupe constitué par la souche BTH101 dont le numéro d'enregistrement de dépôt à la C.N.C.M. est I-2309 et la souche **DHM1** dont le numéro d'enregistrement de dépôt à la C.N.C.M. est I-2310 ;
(c) un milieu permettant la détection de la complémentation choisi dans le groupe constitué par un indicateur ou un milieu sélectif comme milieu minimal supplémenté par du lactose ou du maltose comme unique source de carbone, un milieu avec des antibiotiques, un milieu pour visualiser la fluorescence, la luminescence, un milieu classique, comme le milieu xGAL, et un milieu qui permet le tri grâce à la présence du récepteur de phage.

25. Kit de sélection d'une molécule présentant un intérêt, ledit kit comprenant :
(a) un système d'amplification de signal selon l'une quelconque des revendications 1 à 9 dans lequel la molécule présentant un intérêt est une molécule mutante comparée à la molécule connue de type sauvage ;
(b) un système d'amplification de signal selon l'une quelconque des revendications 1 à 9 dans lequel la molécule présentant un intérêt est la molécule connue de type sauvage, servant de témoin ;
(c) une souche bactérienne carencée en adénylate cyclase endogène choisie dans le groupe constitué par la souche **BTH101** dont le numéro d'enregistrement de dépôt à la C.N.C.M. est I-2309 et la souche **DHM1** dont le numéro d'enregistrement de dépôt à la C.N.C.M. est I-2310 ;
(d) un milieu permettant la détection de la complémentation choisi dans le groupe constitué par un indicateur ou un milieu sélectif comme milieu minimal supplémenté par du lactose ou du maltose comme unique source de carbone, un milieu avec des antibiotiques, un milieu pour visualiser la fluorescence, la luminescence, un milieu classique, comme le milieu xGAL, et un milieu qui permet le tri grâce à la présence du récepteur de phage pour chaque système d'amplification de signal ;
(e) un moyen de détection pour savoir si le système d'amplification du signal avec la molécule mutante est augmenté ou inhibé par rapport au système d'amplification du signal avec le type sauvage.

26. Méthode de criblage d'une substance capable de stimuler ou d'inhiber l'interaction entre un ligand cible et une molécule présentant un intérêt dans laquelle l'activité respectivement stimulante ou inhibitrice est détectée par un système d'amplification de signal selon l'une quelconque des revendications 1 à 9, au moyen de la génération d'une amplification et respectivement, du déclenchement ou de l'abolition de l'activation de la transcription ;
dans laquelle ladite amplification de signal et ladite activation de transcription déclenchée ou abolie sont comparées à celles obtenues avec un système d'amplification de signal identique sans aucune substance ; et
dans laquelle la méthode de criblage pour la substance est réalisée dans une souche d'*E. coli* choisie dans le groupe constitué par la souche **BTH101** dont le numéro d'enregistrement de dépôt à la C.N.C.M. est 1-2309 et la souche **DHM1** dont le numéro d'enregistrement de dépôt à la C.N.C.M. est I-2310.

27. Méthode de criblage d'une substance capable de stimuler ou d'inhiber l'interaction entre un ligand cible et une molécule présentant un intérêt selon la revendication 26, dans laquelle le système d'amplification de signal comprend un système bactérien multi-hybride d'au moins deux fragments distincts d'une enzyme, dont l'activité enzymatique est restaurée par l'interaction entre ladite molécule présentant un intérêt et ledit ligand cible.

28. Méthode de criblage d'une substance capable de stimuler ou d'inhiber l'interaction entre un ligand cible et une molécule présentant un intérêt selon la revendication 26, dans laquelle le système d'amplification de signal comprend un système bactérien multi-hybride d'au moins un premier fragment d'une enzyme et une substance modulante, dont l'activité est restaurée par l'interaction entre ladite molécule présentant un intérêt et ledit ligand cible.

29. Méthode de criblage d'une substance capable de stimuler l'interaction entre un ligand cible et une molécule présentant un intérêt selon l'une quelconque des revendications 26 à 28, dans laquelle le signal d'amplification correspond à la production d'une molécule signalisante.

30. Méthode de criblage d'une substance capable d'inhiber l'interaction entre un ligand cible et une molécule présentant un intérêt selon l'une quelconque des revendications 26 à 28, dans laquelle le signal d'amplification correspondant à la production d'une molécule signalisante est bloqué ou partiellement aboli.

31. Méthode de criblage d'une substance capable de stimuler l'interaction entre un ligand cible et une molécule présentant un intérêt selon l'une quelconque des revendications 26 à 29, dans laquelle l'activation de la transcription conduit à une expression de gène reporteur.

32. Méthode de criblage d'une substance capable d'inhiber l'interaction entre un ligand cible et une molécule présentant un intérêt selon l'une quelconque des revendications 26 à 28 et la revendication 30, dans laquelle l'activation de la transcription conduisant à une expression de gène reporteur est bloquée ou partiellement abolie.

33. Méthode de criblage d'une substance capable de stimuler ou d'inhiber l'interaction entre un ligand cible et une molécule présentant un intérêt selon l'une quelconque des revendications 26 à 32, dans laquelle le ligand cible est choisi dans le groupe constitué par un récepteur, un ligand, un antigène, un anticorps, une protéine se liant à l'ADN, une glycoprotéine et une lipoprotéine.

34. Méthode de criblage d'une substance capable de stimuler ou d'inhiber l'interaction entre un ligand cible et une molécule présentant un intérêt selon l'une quelconque des revendications 26 à 33, dans laquelle la substance est choisie dans le groupe constitué par les protéines, les glycoprotéines, les lipoprotéines et les ligands.

35. Méthode de criblage d'une substance capable de stimuler ou d'inhiber l'interaction entre un ligand cible et une molécule présentant un intérêt selon la revendication 29 ou 30, dans laquelle la molécule signalisante correspond à la synthèse d'AMPc.

36. Méthode de criblage d'une substance capable de stimuler ou d'inhiber l'interaction entre un ligand cible et une molécule présentant un intérêt selon la revendication 29 ou 30, dans laquelle la molécule signalisante correspond à la synthèse de GMPc.

37. Méthode de criblage d'une substance capable de stimuler ou d'inhiber l'interaction entre un ligand cible et une molécule présentant un intérêt selon la revendication 31 ou 32, dans laquelle le gène reporteur est choisi dans le groupe constitué par un gène codant pour un marqueur nutritionnel comme le lactose, le maltose ; un gène conférant une résistance à des antibiotiques comme l'ampicilline, la kanamycine ou la tétracycline ; un gène codant pour une toxine ; un marqueur coloré comme un marqueur fluorescent du type de la protéine fluorescente verte (GFP) ; un gène codant pour des protéines réceptrices de phage ou un fragment de celles-ci comme le récepteur de phage λ, *lamB* et tout autre gène donnant un phénotype pouvant donner lieu à une sélection.

38. Méthode de criblage d'une substance capable de stimuler ou d'inhiber l'interaction entre un ligand cible et une molécule présentant un intérêt selon l'une quelconque des revendications 26 à 37, dans laquelle la molécule présentant un intérêt est une molécule mutante par rapport à la molécule connue du type sauvage et ladite molécule présentant un intérêt est testée pour déterminer sa capacité à interagir avec le ligand cible.

39. Méthode de criblage d'une substance capable de stimuler ou d'inhiber l'interaction entre un ligand cible et une molécule présentant un intérêt selon l'une quelconque des revendications 26 à 37, dans laquelle le criblage est effectué dans la souche **DHM1.**

40. Méthode de criblage d'une substance capable de stimuler ou d'inhiber l'interaction entre un ligand cible et une molécule présentant un intérêt selon l'une quelconque des revendications 26 à 38, dans laquelle le criblage est effectué dans la souche **BTH101**.

41. Kit de criblage d'une substance capable de stimuler ou d'inhiber l'interaction entre un ligand cible et une molécule présentant un intérêt, ledit kit comprenant :
(a) un système d'amplification de signal selon l'une quelconque des revendications 1 à 9 avec la substance capable de stimuler ou d'inhiber l'interaction entre un ligand cible et une molécule présentant un intérêt ;
(b) un système d'amplification de signal selon l'une quelconque des revendications 1 à 9 sans aucune substance, servant de témoin ;
(c) une souche bactérienne carencée en adénylate cyclase endogène choisie dans le groupe constitué par la souche **BTH101** dont le numéro d'enregistrement de dépôt à la C.N.C.M. est I-2309 et la souche **DHM1** dont le numéro d'enregistrement de dépôt à la C.N.C.M. est I-2310 ;
(d) un milieu permettant la détection de la complémentation choisi dans le groupe constitué par une plaque indicatrice ou un milieu sélectif comme milieu minimal supplémenté par du lactose ou du maltose comme unique source de carbone, un milieu avec des antibiotiques, un milieu pour visualiser la fluorescence, un milieu classique, et un milieu qui permet le tri grâce à la présence du récepteur de phage et
(e) un moyen de détection pour savoir si le système d'amplification du signal avec la substance est augmenté
ou inhibé par rapport au système d'amplification du signal sans aucune substance.

42. Système d'amplification de signal selon l'une quelconque des revendications 1 à 9, le système bactérien multi-hybride contenant :
(a) un premier polypeptide chimère correspondant à un premier fragment d'une enzyme ;
(b) un second polypeptide chimère correspondant à un second fragment d'une enzyme ou à une substance modulante capable d'activer ladite enzyme,
(c) une substance capable de stimuler ou d'inhiber l'interaction entre un ligand cible et une molécule présentant un intérêt,
dans lequel le premier fragment est fusionné à une molécule présentant un intérêt et le second fragment ou la molécule modulante est fusionné à un ligand cible, l'activité de l'enzyme étant restaurée par l'interaction entre ladite molécule présentant un intérêt et ledit ligand cible et une amplification de signal étant générée ; et
une souche bactérienne carencée en adénylate cyclase endogène choisie dans le groupe constitué par la souche **BTH101** dont le numéro d'enregistrement de dépôt à la C.N.C.M. est I-2309 et la souche **DHM1** dont le numéro d'enregistrement de dépôt à la C.N.C.M. est I-2310.

43. Souche **BTH101** dont le numéro d'enregistrement de dépôt à la C.N.C.M. est I-2309.

44. Souche **DHM1** dont le numéro d'enregistrement de dépôt à la C.N.C.M. est I-2310.
